# EUROPEAN PATENT APPLICATION

(11) **EP 4 261 224 A1**
(43) Date of publication of application: **18.10.2023**
(21) Application number: 21902709.1
(22) Date of filing: 10.12.2021
(51) Int. Cl.: C07K 16/28, C07K 16/40, C12N 5/10, A61P 35/00, A61K 39/395, A61K 45/06, G01N 33/573, G01N 33/68

(54) **CD73 ANTIGEN-BINDING PROTEIN AND APPLICATION THEREOF**

(30) Priority: 11.12.2020 CN 202011445512
(71) Applicant: Shanghai Huaota Biopharmaceutical Co., Ltd., Shanghai 201203 (CN); Huabo Biopharm (Shanghai) Co., Ltd., Shanghai 201203 (CN)
(72) Inventor: XU, Jingen, Shanghai 201203 (CN); ZHU, Xiangyang, Shanghai 201203 (CN); YU, Haijia, Shanghai 201203 (CN); WEI, Xiaoyue, Shanghai 201203 (CN); TONG, Ling, Shanghai 201203 (CN); CHEN, Shi, Shanghai 201203 (CN)
(74) Representative: Weickmann & Weickmann PartmbB
(86) International application number: PCT/CN2021/136950
(87) International publication number: WO 2022/122004

(57) **Abstract**

Provided is an isolated antigen-binding protein, capable of binding to CD73. The isolated antigen-binding protein contains HCDR3, and the HCDR3 comprises an amino acid sequences as shown in SEQ ID No.:3. Also provided are a preparation method for the antigen-binding protein, and an application of the antigen-binding protein.

## Description

### FIELD OF THE INVENTION

The present application relates to the field of biomedicine, specifically to an antigen-binding protein of CD73 and its application thereof.

### BACKGROUND OF THE INVENTION

CD73, also known as NT5E, is an extracellular 5-nuclease (NT5E) with a molecular weight of about 70Kd that is anchored to the cell surface by glycophosphatidylinositol (GPI) and exists mainly as a dimer. Under normal physiological conditions, CD73 can be expressed in many tissues or organs, such as the liver, large intestine, kidney, spleen, lung, ovary, lymph node and so on.

A large number of pre-clinical studies have shown that CD73 is abnormally overexpressed on a variety of tumor cells, including glioma, breast cancer, melanoma, non-small cell lung cancer, bladder cancer, ovarian cancer, colorectal cancer, and so on. The upregulated expression of CD73 is closely related to the poor prognosis of tumor patients. Therefore, CD73 can be used as a potential target for clinical treatment and prognosis of a variety of tumors.

However, there is currently no antibody or small molecule drug targeting human CD73 available on the market. It is urgent to develop more CD73-targeted drugs to provide new concepts and prospects for the treatment of cancers with abnormal expression of CD73.

### SUMMARY OF THE INVENTION

The present application provides an isolated antigen-binding protein exhibiting one or more desired functional properties, such as its ability to specifically bind to CD73 or a functionally active fragment thereof; its ability to prevent or reduce the activation of CD73; its ability to bind to at least one of the amino acids at positions 143 to 157 or the amino acid residues of a CD73 sequence corresponding thereto, to the amino acids at positions 178 to 191 or a CD73 sequence corresponding thereto, and to the amino acids at positions 381 to 386 or a CD73 sequence corresponding thereto when it is bound to the said CD73; and its ability to inhibit the enzymatic activity of CD73; as well as its ability to induce endocytosis of CD73 on the cell surface. The present application also provides nucleic acid molecules encoding the isolated antigen-binding proteins, expression vectors, host cells and methods of preparing the isolated antigen-binding proteins. The isolated antigen-binding protein described in the present application can be used for the prevention and/or treatment of diseases and/or disorders, such as tumors.

In one aspect, the present application provides an isolated antigen-binding protein comprising HCDR3, wherein said HCDR3 comprises an amino acid sequence as set forth in SEQ ID No.:3. In some embodiments, isolated antigen-binding protein comprises HCDR2, said HCDR2 comprises an amino acid sequence as set forth in SEQ ID No.: 2.

In some embodiments, isolated antigen-binding protein comprises HCDR1, said HCDR1 comprises an amino acid sequence as set forth in SEQ ID No.: 1.

In some embodiments, the isolated antigen-binding protein comprises LCDR3, which comprises an amino acid sequence as set forth in SEQ ID No.: 6.

In some embodiments, the isolated antigen-binding protein comprises LCDR2, which comprises an amino acid sequence as set forth in SEQ ID No.: 5.

In some embodiments, the isolated antigen-binding protein comprises LCDR1, which comprises an amino acid sequence as set forth in SEQ ID No.: 4.

In some embodiments, the isolated antigen-binding protein comprises H-FR1, the C-terminal of which is directly or indirectly linked to the N-terminal of the said HCDR1, and the said H-FR1 comprises an amino acid sequence as set forth in SEQ ID No.: 7, SEQ ID No.: 15, or SEQ ID No.: 19.

In some embodiments, the isolated antigen-binding protein comprises H-FR2, which is located between the said HCDR1 and the said HCDR2, and the said H-FR2 comprises an amino acid sequence as set forth in SEQ ID No.: 8, SEQ ID No.: 16, or SEQ ID No.: 21.

In some embodiments, the isolated antigen-binding protein comprises H-FR3, which is located between the said HCDR2 and the said HCDR3, and the said H-FR3 comprises an amino acid sequence as set forth in SEQ ID No.: 9, SEQ ID No.: 17, SEQ ID No.: 20, or SEQ ID No.: 22. In some embodiments, the isolated antigen-binding protein comprises H-FR4, the N-terminal of which is linked to the C- terminal of the HCDR3, and the said H-FR4 comprises an amino acid sequence as set forth in SEQ ID No.: 10 or SEQ ID No.: 18.

In some embodiments, the isolated antigen-binding protein comprises L-FR1, the C-terminal of which is directly or indirectly linked to the N-terminal of the LCDR1, and the said L-FR1 comprises an amino acid sequence as set forth in SEQ ID No.: 11 or SEQ ID No.: 23.

In some embodiments, the isolated antigen-binding protein comprises L-FR2, which is located between the said LCDR1 and the said LCDR2, and the said L-FR2 comprises an amino acid sequence as set forth in SEQ ID No.: 12 or SEQ ID No.: 24.

In some embodiments, the isolated antigen-binding protein comprises L-FR3, which is located between the said LCDR2 and the said LCDR3, and the said L-FR3 comprises an amino acid sequence as set forth in SEQ ID No.: 13, SEQ ID No.: 25, SEQ ID No.: 27 or SEQ ID No.: 28. In some embodiments, the isolated antigen-binding protein comprises L-FR4, the N-terminal of which is linked to the C- terminal of the LCDR3, and the said L-FR4 comprises an amino acid sequence as set forth in SEQ ID No.: 14 or SEQ ID No.: 26.

In some embodiments, the isolated antigen-binding protein comprises a VH which comprises an amino acid sequence as set forth in SEQ ID No.: 29, SEQ ID No.: 31, SEQ ID No.: 33 or SEQ ID No.: 35.

In some embodiments, the isolated antigen-binding protein comprises a VL which comprises an amino acid sequence as set forth in SEQ ID No.: 30, SEQ ID No.: 32, SEQ ID No.: 34 or SEQ ID No.: 36.

In some embodiments, the isolated antigen-binding protein comprises the VH and VL selected from any of the following groups:
1) The said VH comprises an amino acid sequence as set forth in SEQ ID No.: 29 and the said VL comprises an amino acid sequence as set forth in SEQ ID No.: 30;
2) The said VH comprises an amino acid sequence as set forth in SEQ ID No.: 31 and the said VL comprises an amino acid sequence as set forth in SEQ ID No.: 32;
3) The said VH comprises an amino acid sequence as set forth in SEQ ID No.: 33 and the VL comprises an amino acid sequence as set forth in SEQ ID No.: 34;
4) The said VH comprises an amino acid sequence as set forth in SEQ ID No.: 35 and the said VL comprises an amino acid sequence as set forth in SEQ ID No.: 36;
5) The said VH comprises an amino acid sequence as set forth in SEQ ID No.: 33 and the said VL comprises an amino acid sequence as set forth in SEQ ID No.: 36; and
6) The said VH comprises an amino acid sequence as set forth in SEQ ID No.: 35 and
the said VL comprises an amino acid sequence as set forth in SEQ ID No.: 34.

In some embodiments, the isolated antigen-binding protein comprises a heavy chain constant region of an antibody.

In some embodiments, the said heavy chain constant region of an antibody is derived from a heavy chain constant region of a human antibody.

In some embodiments, the said heavy chain constant region of an antibody is derived from a human IgG heavy chain constant region.

In some embodiments, the said antibody heavy chain constant region is derived from a human IgG1 heavy chain constant region.

In some embodiments, the isolated antigen-binding protein comprises a light chain constant region of an antibody.

In some embodiments, the said light chain constant region of an antibody is derived from a human Ig kappa constant region.

In some embodiments, the isolated antigen-binding protein comprises an antibody or an antigen-binding fragment thereof.

In some embodiments, the said antigen-binding fragments comprise Fab, Fab', Fv fragments, F (ab')₂, F (ab)₂, scFv, di-scFv, and/or dAb.

In some embodiments, the said antibody is selected from one or more kinds in the following group: a monoclonal antibody, a chimeric antibody, a humanized antibody, and a fully humanized antibody.

In some embodiments, the isolated antigen-binding protein is able to specifically bind to CD73 or a functionally active fragment thereof.

In some embodiments, the said CD73 comprises a human CD73 and/or a monkey CD73.

In some embodiments, the isolated antigen-binding protein is able to prevent or reduce the activation of CD73.

In some embodiments, when bound to the said CD73, the isolated antigen-binding protein is able to bind to at least one of the amino acids at positions 143 to 157 as set forth in SEQ ID No.:40 or the amino acid residues of a CD73 sequence corresponding thereto, to the amino acids at positions 178 to 191 as set forth in SEQ ID No.:40 or a CD73 sequence corresponding thereto, and to the amino acids at positions 381 to 386 as set forth in SEQ ID No.:40 or a CD73 sequence corresponding thereto.

In some embodiments, the said amino acids at positions 143-157 or a CD73 sequence corresponding thereto are located in the N-terminal domain of CD73; the said amino acids at positions 178-191 or a CD73 sequence corresponding thereto are located in the N-terminal domain of CD73, and the said amino acids at positions 381-386 or a CD73 sequence corresponding thereto are located in the C-terminal domain of CD73.

In some embodiments, the isolated antigen-binding protein is able to inhibit the enzymatic activity of CD73.

In some embodiments, the isolated antigen-binding protein is able to induce endocytosis of CD73 on the cell surface.

In another aspect, the present application also provides a polypeptide molecule which comprises the isolated antigen-binding protein.

In some embodiments, the said polypeptide molecule comprises a fusion protein.

In another aspect, the present application also provides an isolated nucleic acid molecule which encodes the isolated antigen-binding protein or the said polypeptide molecule.

In another aspect, the present application also provides a vector which comprises the said nucleic acid molecule.

In another aspect, the present application also provides a cell which comprises the said nucleic acid molecule and/or the said vector.

In another aspect, the present application also provides an immunoconjugate which comprises the isolated antigen-binding protein.

In another aspect, the present application also provides a pharmaceutical composition which comprises the isolated antigen-binding protein, the said polypeptide molecule, the said nucleic acid molecule, the said vector the said cell, and/or the said immunoconjugate, and optionally a pharmaceutically acceptable vector.

In another aspect, the present application also provides a method for preparing the isolated antigen-binding protein, which comprises culturing the said cells under conditions that the isolated antigen-binding protein is expressed.

In another aspect, the present application also provides the use of the isolated antigen-binding protein, the said polypeptide molecule, the said nucleic acid molecule, the said vector, the said cell, the said immunoconjugate and/or the said pharmaceutical composition in the preparation of a drug and the use of the said drug in the prevention and/or treatment of a disease and/or disorder.

In another aspect, the present application also provides methods for preventing and/or treating a disease and/or disorder; the said methods may comprise administering to a subject in need thereof the isolated antigen-binding protein of the application, the said polypeptide molecule, the said nucleic acid molecule, the said vector, the said cell, the said immunoconjugate, and/or the said pharmaceutical composition.

In another aspect, the isolated antigen-binding protein of the present application, the said polypeptide molecule, the said nucleic acid molecule, the said vector, the said cell, the said immunoconjugate, and/or the said pharmaceutical composition can be used to prevent and/or treat a disease and/or disorder.

In some embodiments, the said disease and/or disorder is a CD73-mediated disease and/or disorder.

In some embodiments, the said disease and/or disorder comprise tumors.

In some embodiments, the said tumors include solid tumors and/or hematologic tumors .

In some embodiments, the said disease and/or disorder include breast cancer.

In another aspect, the present application also provides a method of detecting CD73 in a sample, which comprises administering the isolated antigen-binding protein, the said polypeptide molecule, the said nucleic acid molecule, the said vector, the said cells, the said immunoconjugate, and/or the said pharmaceutical composition.

In another aspect, the present application also provides a reagent or kit for detecting CD73 in a sample, which comprises the isolated antigen-binding protein, the said polypeptide molecule, the said nucleic acid molecule, the said vector, the said cells, the said immunoconjugate, and/or the said pharmaceutical composition.

In another aspect, the present application also provides the use of the isolated antigen-binding protein, the said polypeptide molecule, the said nucleic acid molecule, the said vector, the said cells, the said immunoconjugate and/or the said pharmaceutical composition in the preparation of a kit for detecting the presence and/or content of CD73 in a sample.

Other aspects and advantages of the present application can be readily appreciated by those skilled in the art based on the detailed description below. Only examples of the present application are shown and described in the detailed description below. As will be appreciated by those skilled in the art, the contents of the present application enable those skilled in the art to make modifications to the disclosed embodiments without departing from the spirit and scope of the invention contemplated in the present application. Accordingly, the descriptions in the drawings and specifications of the present application are only exemplary and not limiting.

### BRIEF DESCRIPTION OF THE DRAWING

The specific features of the invention to which the present application relates are shown in the appended claims. The features and advantages of the present invention related to the present application can be better understood with reference to the following examples and drawings described in detail. A brief description of the drawings is as follows:
Figure 1 shows the binding activity of the said antigen-binding proteins 900581 and 900565 of the present application to the CD73 on the cell surface.
Figure 2 shows the binding activity of the said antigen-binding proteins 900581 and 900694-900698 of the present application to the CD73 on the cell surface.
Figure 3 shows the inhibitory effect of the said antigen-binding proteins 900581 and 900694-900698 of the present application on the enzymatic activity of recombinant CD73.
Figure 4 shows the inhibitory effect of the said antigen-binding proteins 900581 and 900565 of the present application on the enzymatic activity of CD73 on the cell surface.
Figure 5 shows the inhibitory effect of the said antigen-binding proteins 900581, 900697 and 900698 of the present application on the enzymatic activity of CD73 on the cell surface.
Figure 6 shows the efficiency of the antigen-binding protein 900581 described in the present application in inducing endocytosis of CD73 on the cell surface.
Figure 7 shows a structural model of the binding epitope of the said antigen-binding protein of the present application.
Figures 8A-8C show the competitive binding activity of the said antigen-binding proteins of the present application to 900609.
Figure 9 shows the therapeutic effect of the said antigen-binding protein of the present application in treating tumors *in vivo.*
Figure 10 shows the therapeutic effect of the said antigen-binding protein of the present application in treating tumors *in vivo.*

### DETAILED DESCRIPTION

The embodiments of the invention related to the present application are described below using specific examples, and other advantages and effects of the present invention can be readily appreciated by those familiar with the art based on the contents disclosed in the present specification.

### DEFINITIONS OF TERMS

In the present application, the term "CD73" is also referred to as "NT5E", "cluster of differentiation 73", "5 '-nucleotidase (5'-NT)" or "extracellular 5 '-nucleotidase". The scope of the said terms covers the "full length" CD 73, the unprocessed CD73, and any form of CD73 produced by cellular processing. In the present application, the scope of the term "CD73" covers the full-length wild-type CD73 and its variants, fragments, variants, isoforms and homologues thereof. In some embodiments, the said CD73 may comprise human CD73. For example, the sequences related to human CD73 can be found under Uniprot accession number P21589. In the present application, the said CD73 may comprise an amino acid sequence as set forth in SEQ ID No.: 40.

In the present application, the term "isolated" generally refers to those obtained artificially from the natural state. If a "separated" substance or component occurs in nature, it may indicate a change in the natural environment in which it is located, or the substance is isolated from the natural environment, or both situations have occurred. For example, a polynucleotide or polypeptide that has not been isolated is naturally present in some living animals, and the same polynucleotide or polypeptide with high purity isolated from this natural state is considered isolated. The term "isolated" does not exclude the presence of artificial or synthetic substances or the presence of other impure substances that do not affect the activity of the substance.

In the present application, the term "isolated antigen-binding protein" generally refers to a protein with antigen-binding ability that is isolated from its naturally occurring state. This "isolated antigen-binding protein" may comprise an antigen-binding moiety and, optionally, a framework or scaffolding moiety that allows the antigen-binding moiety to adopt a conformation that facilitates the binding of the said antigen-binding moiety to an antigen. The antigen-binding protein may comprise, for example, an antibody-derived protein framework region (FR) or an alternative protein framework region or an artificial framework region with grafted CDRs or a derivative thereof. Such frameworks include, but are not limited to, antibody-derived framework regions comprising mutations introduced, for example, to stabilize the three-dimensional structure of an antigen-binding protein as well as fully synthetic framework regions that comprises, for example, a biocompatible polymer. See, for example, Korndorfer et al., 2003, Proteins: Structure, Function, and Bioinformatics, 53 (1): 121-129 (2003); Roque et al., Biotechnol. Prog, 20: 639-654 (2004). Examples of antigen-binding proteins include, but are not limited to, a human antibody, humanized antibody, chimeric antibody, recombinant antibody; single-chain antibody; bifunctional antibody, trifunctional antibody; tetrafunctional antibody; Fab, Fab', Fv fragments, F (ab')₂, F (ab)₂, scFv, di-scFv, dAb, anti-IgD antibody, anti-IgE antibody, anti-IgM antibody, anti-IgG1 antibody, anti-IgG2 antibody, anti-IgG3 antibody or anti-IgG4 antibody and a fragment thereof. In the present application, the term "CDR" is also referred to as "complementarity determining region" which generally refers to a region in the variable domain of an antibody that is highly variable in sequence and/or may form a structure-defining ring. Typically, antibodies include six CDRs, with three in VH (HCDR1, HCDR2, HCDR3), and three in VL (LCDR1, LCDR2, LCDR3). In some embodiments, naturally occurring camel antibodies that consist only of heavy chains are also capable of maintaining normal and stable function in the absence of light chains. See, for example, Hamers-Casterman et al., Nature 363: 446-448 (1993); Sheriff et al, Nature Struct. Biol. 3: 733-736 (1996). The CDR of an antibody can be determined by multiple coding systems, such as CCG, Kabat, Chothia, IMGT and the combined consideration of Kabat/Chothia, etc. These coding systems are known in the art and can be found, for example, at http://www.bioinf.org.uk/abs/index.html # kabatnum. For example, an amino acid sequence of the said antigen-binding protein can be numbered as per the IMGT numbering scheme (IMGT, the international ImMunoGeneTics information system@imgt.cines.fr; http://imgt.cines.fr; Lefranc et al., 1999, Nucleic Acids Res. 27: 209-212; Ruiz et al., 2000 Nucleic Acids Res. 28: 219-221; Lefranc et al., 2001, Nucleic Acids Res. 29: 207-209; Lefranc et al., 2003, Nucleic Acids Res. 31: 307-310; Lefranc et al., 2005, DevComp Immunol 29: 185-203). For example, the CDR of the said antigen-binding protein can be determined as per the Kabat numbering system (see, for example, Kabat EA & Wu TT (1971) Ann NY AcadSci 190: 382-391 and Kabat EA et al., (1991) Sequences of Proteins of Immunological Interest, Fifth Edition, U.S.Department of Health and Human Services, NIH Publication No. 91-3242). In the present application, the term "FR" generally refers to a more highly conserved moiety of the variable domain of an antibody, which is referred to as a framework region. Typically, the variable domains of the naturally occurring heavy and light chains each contain four FR regions, i.e., four in the VH (H-FR1, H-FR2, H-FR3, and H-FR4), and four in the VL (L-FR1, L-FR2, L-FR3, and L-FR4).

In the present application, the terms "variable domain" and "variable region" can be used interchangeably and they generally refer to a moiety of an antibody's heavy and/or light chain. The variable domains of the heavy chains and light chains can be referred to as "V_{H}" and "V_{L}", respectively (or "VH" and "VL", respectively). These domains are typically the portions with the greatest variation in the antibody (relative to other antibodies of the same type) and these domains also contain antigen-binding sites.

In the present application, the term "variable" generally means that some segments of the variable domain may differ substantially in sequence between different antibodies. The variable domains mediate antigen binding activity and determine the specificity of the binding of a particular antibody to its particular antigen. However, the variability is not uniformly distributed across all the variable domains. The variability is generally focused on three segments in the light and heavy chain variable domains which are called hypervariable regions (CDRs or HVRs). The more highly conserved portion of the variable domain is called the framework region (FR). The variable domains of the naturally occurring heavy and light chains each contain four FR regions, most of which adopt a beta-pleated sheet conformation linked by three CDRs, which form a loop-shaped junction and, in some cases, form a portion of a β-pleated sheeted structure. The CDRs in each chain are held in close proximity by the FR region, and CDRs from the other chain jointly contribute to the formation of the antigen-binding site of the antibody (see Kabat et al, Sequences of Immunological Interest, Fifth Edition, National Institute of Health, Bethesda, Md. (1991)).

In the present application, the term "antibody" generally refers to an immunoglobulin or fragment thereof or derivative thereof, encompassing any polypeptide including the antigen-binding site, regardless of whether it is produced *in vitro* or *in vivo.* This term includes, but is not limited to, polyclonal, monoclonal, monospecific, polyspecific, nonspecific, humanized, single-chain, chimeric, synthetic, recombinant, hybrid, mutated, and grafted antibodies. Unless otherwise modified by the term "intact", such as in "intact antibody," for the purposes of the present invention, the term "antibody" also includes antibody fragments such as Fab, F (ab')₂, Fv, scFv, Fd, dAb and other antibody fragments that retain the antigen binding function (e.g., specifically binding to human CD73). Typically, such fragments should include an antigen-binding domain. The basic 4-chain antibody unit is a heterotetrameric glycoprotein consisting of two identical light (L) chains and two identical heavy (H) chains. The IgM antibody consists of five basic heterotetrameric units and another polypeptide called the J chain and this antibody contains 10 antigen-binding sites, whereas the IgA antibody consists of two to five basic 4-chain units that can be combined with the J chain and polymerized to form a multivalent combination. In the case of IgG, the molecular weight of a 4-chain unit is generally about 150,000 daltons. Each L chain is linked to the H chain by one covalent disulfide bond, whereas the two H chains are linked to each other by one or more disulfide bonds that depend on the H chain isoform. Each H and L chain also has regularly spaced intra-chain disulfide bridge bonds. Each H chain has a variable domain (VH) at the N-terminal which is followed by three constant domains (CH) each for the alpha and gamma chains and four CH domains for the µ and the ε isoform. Each L chain has a variable domain (VL) at the N-terminal and a constant domain at its other terminal. The VL corresponds to the VH, and the CL corresponds to the first constant domain (CH1) of the heavy chain. The specific amino acid residues are thought to form the interface between the light and heavy chain variable domains. VH and VL pair together and form a single antigen-binding site. For the structure and properties of different classes of antibodies, see, for example, Basic and Clinical Immunology, 8th Edition, Daniel P. Sties, Abba I. Terr and Tristram G. Parsolw (eds), Appleton & Lange, Norwalk, Conn., 1994, p. 71 and Chapter 6. The L chain from any vertebrate species can be classified into one of two distinctly different types, eg. κ and λ, based on an amino acid sequence of their constant domains. The immunoglobulins can be classified into different classes or isotypes based on an amino acid sequence of the constant domain of the heavy chain (CH). The currently existing five classes of immunoglobulins namely the IgA, IgD, IgE, IgG and IgM, have heavy chains namedα, δ, ε, γ and µ, respectively.

In the present application, the term "antigen-binding fragment" generally refers to one or more fragments that is able to specifically bind to an antigen (e.g., CD73). In the present application, the antigen binding fragment may include Fab, Fab', F (ab)₂, Fv fragments, F(ab')₂, scFv, di-scFv and/or dAb.

In the present application, the term "Fab" generally refers to an antigen-binding fragment of an antibody. As mentioned above, papain can be used to digest intact antibodies. After the antibody is digested by papain, it produces two identical antigen-binding fragments, i.e, the "Fab" fragment, and the residual "Fc" fragment (i.e. the Fc region, same as above). The Fab fragment may consist of an intact L chain with the variable region of a heavy chain and the first constant region (C_{H}1) of this H chain (V_{H}).

In the present application, the term "Fab' fragment" generally refers to a monovalent antigen-binding fragment of a human monoclonal antibody that is slightly larger than a Fab fragment. For example, the Fab' fragment may include all the light chains, all the heavy chain variable regions, and all or a portion of the first and second constant regions of the heavy chain. For example, the Fab' fragment may also include a portion or all of the 220-330 amino acid residues of the heavy chain.

In the present application, the term "F (ab')₂" generally refers to antibody fragments produced after the digestion of intact antibodies. The F (ab')₂ fragment contains two Fab fragments held together by a disulfide bond and a portion of the hinge region. The F (ab')₂ fragment has the activity of binding to the bivalent antigen and is able to cross-link with the antigen.

In the application, the term "Fv fragment" generally refers to a monovalent antigen-binding fragment of a human monoclonal antibody, including all or a portion of the heavy chain variable region and the light chain variable region, and it lacks the heavy chain constant region and the light chain constant region. The heavy chain variable regions and light chain variable regions comprise, for example, CDRs. For example, an Fv fragment includes all or a portion of the amino-terminal variable region of about 110 amino acids of the heavy chains and light chains. In the present application, the term "scFv" generally refers to a fusion protein consisting of at least an antibody fragment with the variable region of a light chain and at least an antibody fragment with the variable region of a heavy chain, wherein the said light and heavy chain variable regions are contiguous (e.g., linked by the synthetic linker such as a short flexible polypeptide linker) and can be expressed as a single chain polypeptide, and wherein the said scFv retains the specificity of the intact antibody from which it is derived. Unless otherwise specified, the scFv may have the said VL and VH variable regions in any order (e.g., relative to the N-terminal and the C-terminal of the polypeptide) as used in the present application, and the scFv may comprise the VL-linker-VH or may comprise the VH-linker-VL.

In the present application, the term "dAb" generally refers to an antigen-binding fragment that comprises a VH domain and a VL domain, or comprises a VH domain or a VL domain, see, for example, Ward et al. (Nature, 1989 Oct 12; 341 (6242): 544-6); and Holt et al., Trends, Biotechnol., 2003, 21 (11): 484-490, or the published patent applications of Domantis Ltd., such as WO 06/030220 and WO 06/003388.

In the present application, the term "monoclonal antibody" generally refers to a prepared substance of an antibody molecule consisting of a single molecule. Monoclonal antibodies are often highly specific to a single antigen site. Moreover, unlike conventional polyclonal antibody preparations, which typically have different antibodies against different clusters of determinants, each monoclonal antibody is directed against an individual cluster of determinants on an antigen. In addition to their specificity, monoclonal antibodies have the advantage that they can be synthesized in culture with hybridoma cells, thus avoiding the risk of contamination by other immunoglobulins. The modifier "monoclonal" denotes a characteristic of an antibody obtained from a substantially homogeneous antibody population and is not interpreted as requiring the production of an antibody by any particular method. For example, the monoclonal antibodies used in the present application can be prepared in hybridoma cells or using the recombinant DNA technique.

In the present application, the term "chimeric antibody" generally refers to an antibody in which the variable region is derived from one species and the constant region is derived from another species. Typically, the variable regions are derived from antibodies of laboratory animals such as rodents ("parental antibodies"), and the constant regions are derived from human antibodies, such that the resulting chimeric antibodies have a reduced likelihood of eliciting adverse immune responses in human individuals compared to parental (e.g., mouse-derived) antibodies. In the present application, the term "humanized antibody" generally refers to an antibody in which some or all of the amino acids other than those in the CDR regions of a non-human antibody (e.g., a mouse antibody) have been replaced with corresponding amino acids derived from human immunoglobulins. Small amounts of additions, deletions, insertions, substitutions or modifications of amino acids in the CDR regions may also be allowable as long as they still retain the antibody's ability to bind to a particular antigen. The humanized antibody may optionally comprise at least a portion of the constant region of a human immunoglobulin. A "humanized antibody" retains the antigen specificity that is similar to the original antibodies. The "humanized" forms of non-human (e.g., murine) antibodies may minimally contain chimeric antibodies containing sequences derived from non-human immunoglobulins. Under some circumstances, the residues in the CDR region of a human immunoglobulin (recipient's antibody) can be substituted with residues in the CDR region of a non-human species (donor's antibody) (such as a mouse, a rat, a rabbit, or a non-human primate) that has the desired properties, binding affinities, and/or capabilities. Under some circumstances, residues in the FR region of human immunoglobulins can be substituted with corresponding non-human residues. In addition, the humanized antibody may comprise amino acid modifications that are absent either in the recipient's antibody or the donor's antibody. These modifications may be made to further improve the performance of the antibody, such as binding affinity.

The term "fully humanized antibody" generally refers to an antibody that contains only the protein sequence of a human immunoglobulin. A fully humanized antibody may contain the sugar chains of mice if it is produced in mice, mouse cells, or hybridomas derived from mouse cells. Similarly, "mouse antibody" or "rat antibody" refers to an antibody containing only the immunoglobulin sequences of mice or rats, respectively. Fully humanized antibodies can be generated in the human body or transgenic animals having human immunoglobulin germline sequences using the method of bacteriophage display or other molecular biological methods. Exemplary techniques that can be used to manufacture antibodies are described in U.S. Patents with the No.: 6,150,584, 6,458,592 and 6,420,140. Other techniques, such as the use of libraries, are known in the art.

In the present application, the terms "polypeptide molecule", "polypeptide", and "peptide" can be used interchangeably and they generally refer to the polymers of amino acid residues. The term "fusion protein" generally refers to a polypeptide with at least two moieties covalently linked together. Each of the moieties may be polypeptides having different properties. This property may be a biological property, such as *in vitro* or *in vivo* activity. This property can also be a simple chemical or physical property, such as binding to a target molecule or catalysis of a reaction, etc. The two moieties can be directly linked by a single peptide bond or by a peptide linker.

In the present application, the term "nucleic acid molecule" generally refers to nucleotides, deoxyribonucleotides or ribonucleotides of any length in isolated form or isolated or artificially synthesized analogs derived from their natural environment.

In the present application, the term "vector" generally refers to a nucleic acid delivery vehicle into which a polynucleotide encoding a protein can be inserted and the protein can be expressed. The vector can be expressed by transforming, transducing, or transfecting the host cells such that the elements of genetic material it carries are expressed within the host cell. For example, the vector may comprise plasmids, phagemids, cosmids, artificial chromosomes such as yeast artificial chromosomes (YAC), bacterial artificial chromosomes (BAC) or P1-derived artificial chromosomes (PAC) and bacteriophages such as λ bacteriophages or M13 bacteriophages and animal viruses. Animal virus species used as vectors may include retroviruses (including lentiviruses), adenoviruses, adeno-associated viruses, herpesviruses (e.g., herpes simplex virus), poxviruses, baculoviruses, papillomaviruses and papovaviridae (e.g., SV40). A vector may contain a variety of expression-controlling elements, including a promoter sequence, a transcription initiation sequence, an enhancer sequence, a selection element, and a reporter gene. In addition, the vector may also contain a replication initiation site. The vector may also comprise components such as viral particles, liposomes, or protein capsids that facilitate their entry into the cell, but the components are not limited to these substances.

In the present application, the term "cell" generally refers to a single cell, cell line, or cell culture of a person who may, or has been, a recipient of the plasmids or vectors of a subject, which comprises the said nucleic acid molecule of the present invention or the said vector of the present invention. The cells may comprise the progeny of an individual cell. The progeny may not necessarily be identical to the original mother cell (in terms of the morphology of the total DNA complement or the genome) due to naturally occurring, accidental or intentional mutations. The cells may include cells transfected *in vitro* with the said vector of the present application. The cells may be bacterial cells (e.g., *Escherichia coli*), yeast cells, or other eukaryotic cells, such as COS cells, Chinese hamster ovary (CHO) cells, CHO-K1 cells, LNCAP cells, HeLa cells, HEK293 cells, COS-1 cells and NS0 cells. In some embodiments, the cells are mammalian cells. In some embodiments, the mammalian cells are HEK293 cells. In the present application, the term "immunoconjugate" generally refers to a conjugate formed from the conjugation between the said other agents (e.g., a chemotherapeutic agent, a radioactive element, a cell growth inhibitor, and a cytotoxic agent) and the said antibody or an antigen-binding fragment thereof (e.g., covalently linked by a linker molecule), and this conjugate can specifically bind to an antigen on a target cell by the said antibody or an antigen-binding fragment thereof so as to deliver the said other agent into the target cell (e.g., tumor cell).

In the present application, the term "pharmaceutical composition" generally refers to a composition used for the prevention/treatment of a disease or condition. The said pharmaceutical composition may comprise the isolated antigen-binding protein of the present application, the said nucleic acid molecule of the present application, the said vector of the present application, and/or the said cell of the present application, and optionally a pharmaceutically acceptable adjuvant. In addition, the said pharmaceutical composition may also comprise a suitable formulation that contains one or more (pharmaceutically effective) vectors, stabilizers, excipients, diluents, solubilizers, surfactants, emulsifiers and/or preservatives. The acceptable ingredients of the composition are preferably non-toxic to the recipient at the doses and concentrations administered. The pharmaceutical compositions of the present invention include, but are not limited to, liquid, frozen, and lyophilized compositions.

In the present application, the term "pharmaceutically acceptable vector" generally includes a pharmaceutically acceptable vector, excipient, or stabilizer that, at the doses and concentrations utilized, is non-toxic to the cells or mammals exposed thereto. Physiologically acceptable vectors may include, for example, buffers, antioxidants, low-molecular-weight (less than about 10 residues) polypeptides, proteins, hydrophilic polymers, amino acids, monosaccharides, disaccharides and other carbohydrates, chelating agents, sugar alcohols, salt-forming counter ions, such as sodium and/or nonionic surfactants.

In the present application, the term "specific binding" or "specific" generally refers to measurable and reproducible interactions, such as the binding between a target and an antibody, in which the presence of a target can be determined in the presence of a heterogeneous population of molecules, including biomolecules. For example, an antibody that specifically binds to a target (which can be an epitope), may be an antibody that binds to that target with greater binding activity, greater affinity, more easiness, and/or for a greater duration than it binds to other targets. In some embodiments, the antibody specifically binds to an epitope on the protein that is conserved in proteins of different species. In some embodiments, "specific binding" may include, but does not require, exclusive binding.

In the present application, the term "subject" generally refers to a human or non-human animal, including, but not limited to, a cat, dog, horse, pig, cow, sheep, rabbit, mouse, rat, or monkey. The term "tumor" usually refers to any new pathological hyperplasia of tissues. Tumor cells can spread locally or through the bloodstream and lymphatic system to other parts of the body. In the present application, the said tumors may include benign and malignant tumors. In the present application, the said tumor may comprise a solid tumor and/or hematologic malignancy. In the present application, the said tumor may comprise cancer. In the present application, examples of such tumors include, but are not limited to: brain glioma, breast cancer, melanoma, non-small cell lung cancer, bladder cancer, ovarian cancer, and colorectal cancer.

In the present application, the term "activated" generally refers to the conversion from an inactive state to an active one. In some embodiments, "preventing or reducing activation of CD73" may refer to preventing or reducing the conversion of CD73 from a catalytically inactive open conformation to a catalytically active closed conformation.

In the present application, the term "reduction" generally refers to a decrease in the amount and/or extent compared to the original level. For example, the said reducing the activation of CD73 may refer to reducing the activation of CD73 by at least about 0.5-fold, about 1-fold, about 1.5-fold, about 2-fold, about 5-fold, about 10-fold, about 20-fold, about 30-fold, about 40-fold, about 50-fold, about 60-fold, about 70-fold, about 80-fold, about 90-fold, or about 100-fold as compared to that in the absence of the isolated antigen-binding protein.

In the present application, the proteins, polypeptides and/or amino acid sequences involved should also be understood to comprise at least the following substances: the variants or homologues with the same or similar function as the said proteins or polypeptides.

In the present application, the variant may be, for example, a protein or polypeptide that has undergone a substitution, deletion, or addition of one or more amino acids in an amino acid sequence of the said protein and/or the said polypeptide (e.g., an antibody or fragment thereof that specifically binds to the CD73 protein). For example, the functional variant may comprise a protein or polypeptide that has had alterations in the amino acid with at least 1, e.g., 1-30, 1-20, or 1-10, and e.g., 1, 2, 3, 4, or 5 amino acid substitutions, deletions, and/or insertions. The said functional variant may basically retain a biological property of the protein or the polypeptide prior to the alterations (e.g., substitution, deletion, or addition). For example, the functional variant may retain at least 60%, 70%, 80%, 90%, or 100% of the biological activity (e.g., antigen binding capacity) of the protein or the polypeptide prior to the alteration. For example, the said substitution may be conservative.

In the present application, the homologue may be a protein or polypeptide having at least about 85% (e.g., having at least about 85%, about 90%, about 91%, about 92%, about 93%, about 94%, about 95%, about 96%, about 97%, about 98%, about 99% or greater) sequence homology to an amino acid sequence of the protein and/or the polypeptide (e.g., an antibody or fragment thereof that specifically binds to CD73 protein).

In the present application, the said homology generally refers to similarity, resemblance or association between two or more sequences. The "percent sequence homology" can be calculated using the following method: comparing two sequences to be aligned in a comparison window to determine the number of positions in the two sequences where the same nucleic acid bases (e.g., A, T, C, G, I) or the same amino acid residues (e.g., Ala, Pro, Ser, Thr, Gly, Val, Leu, Ile, Phe, Tyr, Trp, Lys, Arg, His, Asp, Glu, Asn, Gln, Cys, and Met) are present so as to obtain the number of matching positions, then divide the number of matching positions by the total number of positions in the comparison window (i.e., window size), and multiply the result by 100 to produce the percent sequence homology. The alignments for the determination of percent sequence homology may be implemented in a variety of ways known in the art, for example, using publicly available computer software such as BLAST, BLAST-2, ALIGN or Megalign (DNASTAR) software. Those skilled in the art may determine the suitable parameters for aligning the sequences, including any algorithm required to achieve maximum alignment within the full-length sequence being compared or within the region of the sequence of interest. The said homology can also be determined by the following methods of FASTA and BLAST. The description of the FASTA algorithm can be found in W. R. Pearson and D. J. Lipman DJ, Improved Tools for Biological Sequence Comparison, Proc. Natl. Acad. Sci.), 85: 2444-2448, 1988; Lipman.D.J., and Pearson.W. R.Rapid and sensitive protein similarity searches. Science, 227: 1435-1441, 1989. A description of the BLAST algorithm can be found in S. Altschul,W. Gish, W. Miller, E. W. Myers, D. and D. Lipman's. J. Basic local alignment search tool," J Molec Biol, 215: 403-410, 1990.

In the present application, the term "including" generally refers to the meaning of including, summarizing, containing, or covering. Under some circumstances, it also means "as" and "consisting of".

In the present application, the term "approximately" generally refers to the variation within a range of 0.5%-10% above or below a specified value, such as variation within a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below a specified value.

### DETAILED DESCRIPTION OF THE INVENTION

### Isolated Antigen-binding Protein

The CDR of an antibody, also referred to as complementarity-determining region, is a portion of the variable region. The amino acid residues in this region may come into contact with an antigen or an antigenic epitope. The CDR of an antibody can be determined by multiple coding systems, such as CCG, Kabat, Chothia, IMGT and the combined consideration of Kabat/Chothia, etc. These coding systems are known in the art and can be found, for example, at http://www.bioinf.org.uk/abs/index.html#kabatnum. Those skilled in the art can identify the CDR regions using different coding systems based on the sequence and structure of the antibody. The CDR regions may vary when different coding systems are used. In the present application, the said CDR comprises CDR sequences that are classified according to any CDR classification method and the variants thereof; the said variants comprise an amino acid sequence of the said CDR that has undergone substitution, deletion and/or addition of one or more amino acids. For example, the substitution, deletion and/or insertion of 1-30, 1-20 or 1-10 amino acids, and for example 1, 2, 3, 4, 5, 6, 7, 8 or 9 amino acids and also the homologues thereof, which may be amino acid sequences having at least about 85% (e.g., having at least about 85%, about 90%, about 91%, about 92%, about 93%, about 94%, about 95%, about 96%, about 97%, about 98%, about 99% or greater) sequence homology to an amino acid sequence of the said CDR. In some embodiments, the CDR is determined by an IMGT numbering scheme.

In one aspect, the application provides an isolated antigen-binding protein that may comprise HCDR3, said HCDR3 may comprise an amino acid sequence as set forth in SEQ ID No.:3.

In the present application, the isolated antigen-binding protein may also comprise HCDR2 which may comprise an amino acid sequence as set forth in SEQ ID No.:2.

In the present application, the isolated antigen-binding protein may also comprise HCDR1 which may comprise an amino acid sequence as set forth in SEQ ID No.: 1.

In the present application, the isolated antigen-binding protein may comprise HCDR3, HCDR2 and HCDR1. For example, the HCDR3 of the isolated antigen-binding protein may comprise an amino acid sequence as set forth in SEQ ID No.:3; the HCDR2 may comprise an amino acid sequence as set forth in SEQ ID No:2, and the HCDR1 may comprise an amino acid sequence as set forth in SEQ ID No.: 1.

In the present application, the isolated antigen-binding protein may comprise LCDR3 which may comprise an amino acid sequence as set forth in SEQ ID No.: 6.

In the present application, the isolated antigen-binding protein may comprise LCDR2 which may comprise an amino acid sequence as set forth in SEQ ID No.:5.

In the present application, the isolated antigen-binding protein may comprise LCDR1 which may comprise an amino acid sequence as set forth in SEQ ID No.:4.

In the present application, the isolated antigen-binding protein may comprise LCDR3, LCDR2 and LCDR1. For example, the LCDR3 of the isolated antigen-binding protein may comprise an amino acid sequence as set forth in SEQ ID No.: 6; the LCDR2 may comprise an amino acid sequence as set forth in SEQ ID No: 5, and the LCDR1 may comprise an amino acid sequence as set forth in SEQ ID No.: 4.

In the present application, the isolated antigen-binding protein may comprise HCDR3, HCDR2, HCDR1, LCDR3, LCDR2 and LCDR1. For example, the isolated antigen-binding protein HCDR3 described in the present application may comprise an amino acid sequence as set forth in SEQ ID No.: 3; the HCDR2 may comprise an amino acid sequence as set forth in SEQ ID No.:2; the HCDR1 may comprise an amino acid sequence as set forth in SEQ ID No.: 1; the LCDR3 may comprise an amino acid sequence as set forth in SEQ ID No.: 6; the LCDR2 may comprise an amino acid sequence as set forth in SEQ ID No.:5, and the LCDR1 may comprise an amino acid sequence as set forth in SEQ ID No.: 4.

In the present application, the isolated antigen-binding protein may comprise H-FR1; the said C terminal of which may be directly or indirectly linked to the N terminus of the HCDR1, and the said H-FR1 may comprise an amino acid sequence as set forth in SEQ ID No.:7, SEQ ID No.: 15, or SEQ ID No.: 19.

In the present application, the isolated antigen-binding protein may comprise H-FR2, the said H-FR2 may be located between the said HCDR1 and the said HCDR2, and the said H-FR2 may comprise an amino acid sequence as set forth in SEQ ID No.: 8, SEQ ID No.: 16, or SEQ ID No.:21.

In the present application, the isolated antigen-binding protein may comprise H-FR3, which may be located between the said HCDR2 and the said HCDR3, and the said H-FR3 may comprise an amino acid sequence as set forth in SEQ ID No.: 9, SEQ ID No.: 17, SEQ ID No.:20, or SEQ ID No.: 22.

In the present application, the isolated antigen-binding protein may comprise H-FR4, the N-terminal of which may be linked to the C-terminal of the said HCDR3, and the said H-FR4 may comprise an amino acid sequence as set forth in SEQ ID No.: 10 or SEQ ID No.: 18.

In the present application, the said antigen-binding protein may comprise H-FR1, H-FR2, H-FR3 and H-FR4. For example, the said H-FR1, H-FR2, H-FR3 and H-FR4 of the isolated antigen-binding protein may comprise, in sequence, an amino acid sequences as set forth in SEQ ID No.: 7, SEQ ID No.: 8, SEQ ID No.: 9 and SEQ ID No.: 10, respectively. For example, the H-FR1, H-FR2, H-FR3 and H-FR4 of the isolated antigen-binding protein may comprise, in sequence, an amino acid sequences as set forth in SEQ ID No.: 15, SEQ ID No.:16, SEQ ID No.:17 and SEQ ID No.: 18, respectively. For example, the H-FR1, H-FR2, H-FR3 and H-FR4 of the isolated antigen-binding protein may comprise, in sequence, an amino acid sequences as set forth in SEQ ID No.: 19, SEQ ID No.: 16, SEQ ID No.:20 and SEQ ID No.: 18, respectively. For example, the H-FR1, H-FR2, H-FR3 and H-FR4 of the isolated antigen-binding protein may comprise, in sequence, an amino acid sequences as set forth in SEQ ID No.: 19, SEQ ID No.:21, SEQ ID No.:22 and SEQ ID No.: 18, respectively.

In the present application, the isolated antigen-binding protein may comprise L-FR1, the C-terminal of which may be directly or indirectly linked to the N-terminal of the said LCDR1, and the said L-FR1 may comprise an amino acid sequence as set forth in SEQ ID No.: 11 or SEQ ID No.:23.

In the present application, the isolated antigen-binding protein may comprise L-FR2, which may be located between the said LCDR1 and the said LCDR2, and the said L-FR2 may comprise an amino acid sequence as set forth in SEQ ID No.:12 or SEQ ID No.:24.

In the present application, the isolated antigen-binding protein may comprise L-FR3, which may be located between the said LCDR2 and the said LCDR3, and the said L-FR3 may comprise an amino acid sequence as set forth in SEQ ID No.:13, SEQ ID No.:25, SEQ ID No.:27 or SEQ ID No.:28.

In the present application, the isolated antigen-binding protein may comprise L-FR4, the N-terminal of which may be linked to the C-terminal of the said LCDR3, and the said L-FR4 may comprise an amino acid sequence as set forth in SEQ ID No.:14 or SEQ ID No.:26.

In the present application, the isolated antigen-binding protein may comprise L-FR1, L-FR2, L-FR3 and L-FR4. For example, the L-FR1, L-FR2, L-FR3 and L-FR4 of the isolated antigen-binding protein may comprise, in sequence, an amino acid sequences as set forth in SEQ ID No.:11, SEQ ID No.:12, SEQ ID No.:13 and SEQ ID No.:14, respectively. For example, the L-FR1, L-FR2, L-FR3 and L-FR4 of the isolated antigen-binding protein may comprise, in sequence, an amino acid sequences as set forth in SEQ ID No.:23, SEQ ID No.:24, SEQ ID No.:25 and SEQ ID No.:26, respectively. For example, the L-FR1, L-FR2, L-FR3 and L-FR4 of the isolated antigen-binding protein may comprise, in sequence, an amino acid sequences as set forth in SEQ ID No.:23, SEQ ID No.:24, SEQ ID No.:27 and SEQ ID No.:26, respectively. For example, the L-FR1, L-FR2, L-FR3 and L-FR4 of the isolated antigen-binding protein may comprise, in sequence, an amino acid sequences as set forth in SEQ ID No.:23, SEQ ID No.:24, SEQ ID No.: 28 and SEQ ID No.:26, respectively.

In the present application, the isolated antigen-binding protein may comprise a VH which may comprise an amino acid sequence as set forth in SEQ ID No.:29, SEQ ID No.:31, SEQ ID No.:33 or SEQ ID No.:35.

In the present application, the isolated antigen-binding protein may comprise a VL which may comprise an amino acid sequence as set forth in SEQ ID No.:30, SEQ ID No.:32, SEQ ID No.:34 or SEQ ID No.:36.

In the present application, the isolated antigen-binding protein may comprise the said VH and VL. In some embodiments, the said VH may comprise an amino acid sequence as set forth in SEQ ID No.:29, and the VL may comprise the said amino acid sequence as set forth in SEQ ID No.:30. In some embodiments, the said VH may comprise an amino acid sequence as set forth in SEQ ID No.:31, and the VL may comprise the said amino acid sequence as set forth in SEQ ID No.:32. In some embodiments, the said VH may comprise an amino acid sequence as set forth in SEQ ID No.:33, and the VL may comprise the said amino acid sequence as set forth in SEQ ID No.:34. In some embodiments, the said VH may comprise an amino acid sequence as set forth in SEQ ID No.:35, and the VL may comprise the said amino acid sequence as set forth in SEQ ID No.:36. In some embodiments, the said VH may comprise an amino acid sequence as set forth in SEQ ID No.:33, and the VL may comprise the said amino acid sequence as set forth in SEQ ID No.:36. In some embodiments, the said VH may comprise an amino acid sequence as set forth in SEQ ID No. :35, and the VL may comprise the said amino acid sequence as set forth in SEQ ID No.:34.

In the present application, the isolated antigen-binding protein may comprise at least one CDR in the said VH of the present application. In the present application, the isolated antigen-binding protein may comprise at least one CDR in the said VL of the present application. The said CDR can be classified according to any classification method.

In the present application, the isolated antigen-binding protein may comprise the said HCDR1, HCDR2 and HCDR3 in the said VH of the present application. The said VH may comprise an amino acid sequence as set forth in SEQ ID No.:29, SEQ ID No.:31, SEQ ID No.:33 or SEQ ID No.:35.

In the present application, the isolated antigen-binding protein may comprise LCDR1, LCDR2 and LCDR3 in the said VL of the present application. The said VL may comprise an amino acid sequence as set forth in SEQ ID No.:30, SEQ ID No.:32, SEQ ID No.:34 or SEQ ID No.:36. In the present application, the isolated antigen-binding protein may include the heavy chain constant region of the antibody. The said heavy chain constant region of the antibody may be derived from a human IgG heavy chain constant region. In some embodiments, the isolated antigen-binding protein may comprise a heavy chain constant region of the antibody, and the said heavy chain constant region of the antibody may be derived from a human IgG1 heavy chain constant region.

In the present application, the isolated antigen-binding protein may comprise the light chain constant region of the antibody. The light chain constant region of the antibody may be derived from a human Ig kappa constant region.

In the present application, the isolated antigen-binding protein may comprise an antibody or an antigen binding fragment thereof.

In some embodiments, the said antigen-binding fragments may comprise Fab, Fab', Fv fragments, F (ab')₂, F (ab)₂, scFv, di-scFv, and/or dAb.

In some embodiments, the said antibody may comprise a monoclonal antibody, a chimeric antibody, a humanized antibody, and/or a fully humanized antibody.

For example, the VH of the said chimeric antibody may comprise an amino acid sequence as set forth in SEQ ID No.:29, and the VL of the said chimeric antibody may comprise an amino acid sequence as set forth in SEQ ID No.:30.

For example, the VH of the said humanized antibody may comprise an amino acid sequence as set forth in SEQ ID No.:31, and the VL of the said humanized antibody may comprise an amino acid sequence as set forth in SEQ ID No.:32.

For example, the VH of the said humanized antibody may comprise an amino acid sequence as set forth in SEQ ID No.:33, and the VL of the said humanized antibody may comprise an amino acid sequence as set forth in SEQ ID No.:34.

For example, the VH of the said humanized antibody may comprise an amino acid sequence as set forth in SEQ ID No. :35, and the VL of the said humanized antibody may comprise an amino acid sequence as set forth in SEQ ID No.:36.

For example, the VH of the said humanized antibody may comprise an amino acid sequence as set forth in SEQ ID No.:33, and the VL of the said humanized antibody may comprise an amino acid sequence as set forth in SEQ ID No.:36.

For example, the VH of the said humanized antibody may comprise an amino acid sequence as set forth in SEQ ID No. :35, and the VL of the said humanized antibody may comprise an amino acid sequence as set forth in SEQ ID No.:34.

Furthermore, it should be stated that the isolated antigen-binding proteins of the present application may comprise the heavy and/or light chain sequences in which one or more conserved sequences are modified. The so-called "conserved sequence modification" is an amino acid modification that does not significantly affect or alter the antibody's binding property. Such conservative modifications include amino acid substitutions, additions and deletions. Modifications can be introduced into the isolated antigen-binding proteins of the present application using standard techniques known in the art, such as point mutations and PCR-mediated mutations. Conservative amino acid substitution refers to the replacement of an amino acid residue using another amino acid residue with a similar side chain. Groups of amino acid residues with similar side chains are known within the field. These groups of amino acid residues include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), β-branched side chains (e.g., threonine, valine, isoleucine), and aromatic side chains (e.g., tyrosine, phenylalanine, tryptophan, histidine). In some embodiments, one or more amino acid residues in the CDR region of the isolated antigen-binding protein of the present application can be substituted with other amino acid residues of the side chains in the same group. Those skilled in the art know that some modifications to the conservative sequence will not eliminate the antigen binding capacity, see, for example, Brummell et al., (1993) Biochem 32:1180-8; de Wildt et al., (1997) Prot. Eng. 10:835-41; Komissarov et al., (1997) J. Biol. Chem. 272:26864-26870; Hall et al., (1992) J. Immunol. 149:1605-12; Kelley and O' Connell (1993) Biochem. 32:6862-35; Adib-Conquy et al., (1998) Int. Immunol. 10:341-6 and Beers et al., (2000) Clin. Can. Res. 6:2835-43.

The said anti-CD73 antigen-binding proteins of the present application can be identified, screened or characterized by various assays known in the art.

For example, the antigen-binding activity of the antigen-binding protein or fusion protein of the present application can be tested by known methods such as the enzyme-linked immunosorbent assay (ELISA), immunoblotting (e.g., Western blot), flow cytometry, e.g., FACS), immunohistochemistry and immunofluorescence, etc.

In the present application, the isolated antigen-binding protein is able to specifically bind to CD73 or a functionally active fragment thereof.

In some embodiments, the affinity of the isolated antigen-binding proteins can be screened using the Biacore technique. The isolated antigen-binding protein may bind to the CD73 protein with a KD of ≤5 × 10⁻⁹ M, for example, the said antigen-binding protein of the present application may bind to CD73 with a KD of less than about 4 × 10⁻⁹ M, less than about 3 × 10⁻⁹ M, less than about 2 × 10⁻⁹ M, less than about 1 × 10⁻⁹ M, less than about 1 × 10⁻⁹ M, less than about 7 × 10⁻¹⁰ M, less than about 6 × 10⁻¹⁰ M, less than about 5 × 10⁻¹⁰ M, less than about 4 × 10⁻¹⁰ M, less than about 3 × 10⁻¹⁰ M, less than about 2 × 10⁻¹⁰ M, less than about 1 × 10⁻¹¹ M, less than about 5 × 10⁻¹¹ M and less than about 2 × 10⁻¹¹ M. In some embodiments, the present application also includes determining the binding activity of an antigen-binding protein to an antigen using the FACS method. For example, the said antigen-binding protein of the present application can bind to human CD73 on the cell surface with an EC₅₀ value of ≤ about 0.5 µg/mL, ≤ about 0.4 µg/mL, ≤ about 0.3 µg/mL, ≤ about 0.2 µg/mL, and ≤ about 0.1 µg/mL. For example, the said antigen-binding protein of the present application can bind to human CD73 on the cell surface with an EC₅₀ value of ≤ about 0.4 µg/mL, ≤ about 0.3 µg/mL, ≤ about 0.2 µg/mL, and ≤ about 0.1 µg/mL.

In some embodiments, the isolated antigen-binding protein can bind to CD73. Under some circumstances, the said antigen-binding proteins of the present application may also cross-react with the CD73 of monkeys, for example, as detected by FACS. In the present application, "cross-reactivity" generally refers to the ability of an antibody to react with homologous proteins derived from other species.

In some embodiments, the isolated antigen-binding protein of the present application does not bind to the CD73 of mice and/or rats.

For example, the potency can be determined by ELISA. For example, the binding activity or functional activity of the isolated antigen-binding protein can be detected using FACS. For example, the binding affinity of the isolated antigen-binding protein can be detected.

In the present application, the isolated antigen-binding protein is able to prevent or reduce the activation of CD73. For example, the isolated antigen-binding protein is able to prevent or reduce the conversion of CD73 from a catalytically inactive open conformation to a catalytically active closed conformation. For example, the isolated antigen-binding protein is able to block the CD73 catalytic center, thereby inhibiting the enzymatic activity of CD73.

In some embodiments, the binding epitopes of the isolated antigen-binding protein can be determined by the hydrogen-deuterium exchange mass spectrometry (HDX-MS) method. In some embodiments, the isolated antigen-binding protein has competitive binding activity against the CPI-006 antibody (Corvus).

In the present application, when bound to the said CD73, the isolated antigen-binding protein may bind to at least one amino acid residue in the 143-NIKAKGPLASQISGL-157 region (SEQ ID No.: 37) of the N-terminal domain of CD73 or a corresponding sequence thereto, the 178-SKETPFLSNPGTNL-191 region (SEQ ID No.: 38) of the N-terminal domain of CD73 or a corresponding sequence thereto, and the 381-WNHVSM-386 region (SEQ ID No.: 39) of the C-terminal domain of CD73 or a corresponding sequence thereto.

In the present application, "CD73 sequence corresponding thereto" may refer to an amino acid sequence at the corresponding position in its homologue (or the CD73 from other species).

In the present application, the isolated antigen-binding protein is able to inhibit the enzymatic activity of CD73. For example, the isolated antigen-binding protein may have an inhibitory effect on the enzymatic activity of recombinant CD73. For example, the isolated antigen-binding protein may have an inhibitory effect on the enzymatic activity of human CD73. The said enzyme activity in the present application can be detected by any method commonly used in the art. The detection of the said enzyme activity may include the following method: mix CD73 antigen-binding protein with recombinant CD73 protein, and the mixture is added with AMP and ATP, and the CellTiter-Glo^{®} substrate is added to the CellTiter-Glo^{®} buffer; the mixture is mixed well and equilibrated to room temperature for reaction, then the detection is performed at the full wavelength. For example, the isolated antigen-binding protein can inhibit the enzymatic activity of the recombinant CD73 protein with an EC₅₀ value of ≤ about 0.8 µg/mL, ≤ about 0.7 µg/mL, ≤ about 0.6 µg/mL, ≤ about 0.5 µg/mL, ≤ about 0.4 µg/mL, ≤ about 0.3 µg/mL and ≤ about 0.2 µg/mL. For example, the isolated antigen-binding protein can inhibit the enzymatic activity of the CD73 protein on the cell surface with an EC₅₀ value of ≤ about 6 µg/mL, ≤ about 5 µg/mL, ≤ about 4 µg/mL, ≤ about 3 µg/mL, ≤ about 2 µg/mL, ≤ about 1 µg/mL and ≤ about 0.5 µg/mL.

In the present application, the isolated antigen-binding protein is able to induce endocytosis of CD73 on the cell surface. In some embodiments, the endocytosis of the protein can be determined by the fluorescent labeling method. For example, the efficiency with which the said antigen-binding protein induces endocytosis of CD73 on the cell surface can be obtained using the flow cytometry method to detect the fluorescence intensity of the cells. Under some circumstances, the isolated antigen-binding protein can induce endocytosis of CD73 with an efficiency of ≥about 50%.

### Polypeptide molecules, nucleic acid molecules, vectors, cells, immunoconjugates and pharmaceutical compositions

In another aspect, the present application provides a polypeptide molecule which may comprise the isolated antigen-binding protein of the present application.

In some embodiments, the said polypeptide molecule may comprise a fusion protein. In some embodiments, the said polypeptide molecule can be a fusion protein.

In another aspect, the present application provides isolated nucleic acid molecules that may encode the isolated antigen-binding proteins of the present application. For example, it can be generated or synthesized by: i) amplified *in vitro,* such as amplified by the polymerase chain reaction (PCR) ; ii) produced by clonal recombination; iii) purified, e.g., by enzyme digestion and isolated and fractionated by gel electrophoresis; or iv) synthesized, e.g., by chemical synthesis.

In another aspect, the present application provides a vector which may comprise the said nucleic acid molecule of the present application. In addition, the said vector may comprise other genes, such as the marker genes that allow for the selection of the vector in the appropriate host cells and under appropriate conditions. In addition, the said vector may also comprise the expression control elements that allow for the proper expression of the coding region in an appropriate host. Such control elements are well known to those skilled in the art and they may include, for example, promoters, ribosome binding sites, enhancers, and other control elements that regulate gene transcription or mRNA translation, among others. The said vectors can be expressed by transforming, transducing, or transfecting the host cells such that the elements of genetic material they carry are expressed within the host cells. The said vectors may include, for example, plasmids, cosmids, viruses, bacteriophages, or other vectors commonly used in, for example, genetic engineering. For example, the said vector is an expression vector. In addition, the said vector may also include, but not limited to components that can assist in its entry into the cell, such as viral particles, liposomes or protein capsids.

In another aspect, the present application provides a cell which may comprise the said nucleic acid molecule or the said vector of the present application. In some embodiments, each type of host cell or each host cell may comprise one or one of the said nucleic acid molecules or vectors of the present application. In some embodiments, each type of host cell or each host cell may comprise multiple (e.g., 2 or more) or multiple types (e.g., 2 or more) of the said nucleic acid molecules or vectors of the present application. For example, the said vectors of the present application may be introduced into the host cell, for example, a eukaryotic cell, such as a cell derived from a plant, a fungal or yeast cell or the like. In some embodiments, the said cells may be bacterial cells (e.g., *Escherichia coli*), yeast cells, or other eukaryotic cells, such as COS cells, Chinese Hamster Ovary (CHO) cells, CHO-K1 cells, LNCAP cells, HeLa cells, 293T cells, COS-1 cells, SP2/0 cells, NS0 cells, or myeloma cells. The said vectors of the present application can be introduced into the host cells by methods known in the art, such as electroporation, lipofectine transfection, lipofectamin transfection, and the like.

In another aspect, the present application also provides an immunoconjugate which may comprise the isolated antigen-binding protein of the present application.

In some embodiments, the isolated antigen-binding protein or fragment thereof of the present application may be linked to another reagent, such as a chemotherapeutic reagent, toxin, immunotherapeutic agent, imaging probe, spectroscopic probe, or the like. This linking may be achieved via one or more covalent bonds, or non-covalent interactions, which may include chelation. A variety of linkers may be used (the said linkers may be known in the art) to form the immunoconjugate. In addition, the immunoconjugate may be provided in the form of a fusion protein, which may be expressed by the polynucleotide encoding the immunoconjugate. The said immunoconjugate may further comprise, for example, an antibody-drug conjugate (ADC). Appropriate drugs may include cytotoxins, alkylating agents, DNA minor groove binding molecules, DNA intercalators, DNA cross-linking agents, histone deacetylase inhibitors, nuclear export inhibitors, proteasome inhibitors, inhibitors of topoisomerases I or II, heat shock protein inhibitors, tyrosine kinase inhibitors, antibiotics, and antimitotic agents. In the ADC, the antibody and the therapeutic agent can be cross-linked by a cleavable linker, such as a peptide linker, a disulfide linker, or a hydrazone linker.

In another aspect, the present application also provides a pharmaceutical composition which may comprise an isolated antigen-binding protein of the present application, a polypeptide molecule of the application, an immunoconjugate of the application, a nucleic acid molecule of the application, a vector of the application, and/or a cell of the application, and optionally a pharmaceutically acceptable vector.

In some embodiments, the said pharmaceutical composition may also comprise an appropriate formulation that contains one or more (pharmaceutically effective) adjuvants, stabilizers, excipients, diluents, solubilizers, surfactants, emulsifiers and/or preservatives. The acceptable ingredients of the composition are preferably non-toxic to the recipient at the doses and concentrations administered. The pharmaceutical compositions of the present invention include, but are not limited to, liquid, frozen, and lyophilized compositions.

In some embodiments, the pharmaceutical compositions may also contain more than one active compound, typically those active compounds having complementary activities that do not adversely affect each other. The type and effective amount of such drugs may depend, for example, on the amount and type of antagonist present in the formulation, as well as the clinical parameters of the subjects.

In some embodiments, the said pharmaceutically acceptable vector may include any or all of the solvents, dispersion media, coatings, isotonizing agents, and absorption delaying agents that are compatible with the administered drug, and the said pharmaceutically acceptable vector is generally safe and non-toxic.

In some embodiments, the route of administration of the said pharmaceutical composition may include parenteral, percutaneous, intraluminal, intraarterial, intrathecal, and/or intranasal administration or direct injection into the tissue. For example, the said pharmaceutical composition may be administered to a patient or a subject via infusion or injection. In some embodiments, the administration of the pharmaceutical composition may be carried out via different routes, such as intravenous, intraperitoneal, subcutaneous, intramuscular, topical, or intradermal administration. In some embodiments, the pharmaceutical composition may be administered non-intermittently. The said non-intermittent (or continuous) administration can be achieved by using a small pump system worn by the patient to measure the therapeutic agent delivered into the body of the patient, as described in WO2015/036583.

### Preparation Method

In another aspect, the present application provides methods for preparing the said antigen-binding proteins. The said method may comprise culturing the said host cell of the present application under the conditions that the antigen-binding protein is expressed. For example, this can be achieved by the use of appropriate culture medium, appropriate temperature and culturing time, which are known to the ordinary technicians in the art.

Any method suitable for producing monoclonal antibodies can be used to produce the antigen-binding proteins of the present application. For example, animals can be immunized with ligated or naturally occurring CD73 or fragments thereof. Appropriate immunization methods can be used, including adjuvants, immunostimulants, and repeated booster shots, and one or more routes can be used. For example, a hybridoma preparation method can be used to allow the obtained spleen cells from immunized mice to fuse with the SP2/0 myeloma cells, and then hybridoma cell strains can be screened out by HAT.

Any appropriate form of CD73 can be used as the immunogen (antigen) to generate non-human antibodies directed against CD73 and screen for the biological activity of the said antibodies. For example, the eliciting immunogen may be full-length mature human CD73, including native homodimers, or peptides that contain single/multiple epitopes. The immunogens may be used alone, or in combination with one or more immunogenicity enhancers known in the art. The chimeric human antibody may be selected from any type of immunoglobulins, including IgM, IgD, IgG, IgA and IgE. In the present application, the antibody may be an IgG antibody and the IgG1 subtype may be used. Optimization of the essential constant domain sequences can be achieved by screening antibodies with the biological assay described in the examples below to produce the desired biological activity. Likewise, any type of light chain may be used in the compounds and methods of the present application. For example, κ chains or variants thereof may be used in the compounds and methods of the present application.

### Methods and Uses

In another aspect, the present application provides the use of the isolated antigen-binding protein, the said polypeptide molecule, the said nucleic acid molecule, the said vector, the said cell, the said immunoconjugate and/or the said pharmaceutical composition in the preparation of a drug and the use of the said drug in the prevention and/or treatment of a disease and/or disorder.

In another aspect, the present application also provides methods for preventing and/or treating a disease and/or disorder; the said methods may comprise administering to a subject in need thereof the isolated antigen-binding protein of the application, the said polypeptide molecule, the said nucleic acid molecule, the said vector, the said cell, the said immunoconjugate, and/or the said pharmaceutical composition.

In the present application, the said use can be carried out by different modes, such as intravenous, intratumoral, intraperitoneal, subcutaneous, intramuscular, topical or intradermal administration.

In another aspect, the isolated antigen-binding protein of the present application, the said polypeptide molecule, the said nucleic acid molecule, the said vector, the said cell, the said immunoconjugate, and/or the said pharmaceutical composition can be used to prevent and/or treat a disease and/or disorder.

In the present application, the said disease and/or disorder may be a CD73-mediated disease and/or disorder.

In the present application, the disease and/or disorder comprise tumors.

In the present application, the said tumor comprises a solid tumor and/or hematologic tumor . In the present application, the said disease and/or disorder comprise breast cancer.

In another aspect, the present application also provides a method of detecting CD73 in a sample, and the said method comprises administering the isolated antigen-binding protein, the said polypeptide molecule, the said nucleic acid molecule, the said vector, the said cells, the said immunoconjugate, and/or the said pharmaceutical composition.

Under some circumstances, the said method for detecting CD73 in a sample is an *in vitro* method. Under some circumstances, the said method for detecting CD73 in a sample is for non-therapeutic purposes. Under some circumstances, the method for detecting CD73 in a sample is not a diagnostic method.

In another aspect, the present application also provides a reagent or kit for detecting CD73 in a sample, which comprises the isolated antigen-binding protein, the said polypeptide molecule, the said nucleic acid molecule, the said vector, the said cells, the said immunoconjugate, and/or the said pharmaceutical composition.

In another aspect, the present application also provides the use of the isolated antigen-binding protein, the said polypeptide molecule, the said nucleic acid molecule, the said vector, the said cells, the said immunoconjugate and/or the said pharmaceutical composition in the preparation of a kit for detecting the presence and/or content of CD73 in a sample.

For the purpose of not to be limited by any theory, the examples provided below are merely intended to elaborate on the fusion proteins, methods for preparation and uses described in the present application, instead of intending to limit the scope of the present invention.

### Examples

### Example 1 Method for Preparation of Murine Antigen Binding Protein against Human CD73

1.1 Preparation of Hybridoma Cells for Producing Murine Antigen Binding Proteins The method for preparing murine monoclonal antigen-binding proteins employed the hybridoma preparation technique invented by Kohler and Milstein in 1975 (Nature, 1975, 256: 495-497). First, human CD73 His-tagged protein (Met 1-Lys 547) was used as the immunizing antigen to immunize multiple BALB/c and CD1 mice via subcutaneous injection at multiple sites and part of the proteins were used in combination with Freund's Adjuvant, Complete (Sigma Cat No. F5881, synonym: FCA) and Freund's Adjuvant, Incomplete (Sigma Cat No. F5506, synonym: FICA), and the other part of the proteins were used in combination with Quick Antibody-Mouse 5w (Booron cat no. KX0210041) as the adjuvants. After three shots of immunization, the serum titers were detected by ELISA, and the binding activity and functional activity were detected by FACS. Finally, the best mice were selected to obtain spleen cells, and then the cells were mixed with the SP2/0 myeloma cells for fusion. After the hybridoma cell lines were selected by HAT, the cell culture supernatant was harvested and the FACS method was used to screen for monoclonal hybridoma cell lines that can specifically bind to human CD73 and monkey CD73, as well as the monoclonal cell lines that can inhibit CD73 enzymatic activity; then the screened monoclonal cell lines were screened for affinity by Biacore, and finally, the monoclonal hybridoma cell lines expressing human CD73 antigen-binding protein were obtained for sequence analysis. See Table 1 for the list of screening data.

**Table 1 Screening of Hybridoma**

| **Clone No.** | **Binding activity assay** | | **Functional activity** | **Affinity (KD)** |
|---|---|---|---|---|
| | **Human CD73 (MFI value)** | **Monkey CD73 (MFI value)** | | |
| 3A9 | 2421 | 1221 | 15.06 | 7.84E-10 |
| 115B10 | 6387 | 3266 | 17.88 | 2.94E-09 |
| 14E10 | 5429 | 2413 | 25.91 | 3.61E-09 |
| 36B3 | 5095 | 2000 | 17.01 | 6.97E-10 |
| 47F6 | 5406 | 2215 | 31.73 | 2.08E-09 |
| 72A4 | 5109 | 2218 | 22.09 | 3.15E-09 |
| 100G9 | 5161 | 2277 | 17.55 | 3.09E-09 |
| 124A9 | 5031 | 3116 | 9.91 | 2.16E-09 |
| 125A7 | 5628 | 2566 | 9.19 | 2.97E-09 |
| 127B3 | 4505 | 2044 | 23.81 | 1.96E-09 |
| 132C5 | 5137 | 2778 | 22.33 | 4.38E-09 |
| 144A9 | 5925 | 2900 | 11.29 | 3.15E-09 |
| 148F9 | 5325 | 2551 | 11.96 | 1.88E-09 |
| 151A6 | 3402 | 1318 | 14.63 | 2.32E-09 |
| 160B5 | 5133 | 2761 | 18.56 | 1.85E-09 |

After high throughput screening, the finally obtained monoclonal hybridoma cell lines had high affinity, and could bind to human CD73 and monkey CD73.

### Example 2 Sequencing of Anti-CD73 Antigen Binding Protein Variable Region Gene and Preparation of Each Antigen Binding Protein

### 2.1 Sequencing and Cloning of Variable Region Genes of Antigen-binding Proteins in Hybridoma Cells

Based on the principle of the 5' RACE technique of TAKARA, the cDNA sequence of the variable region of the mice antigen-binding protein expressed by hybridoma cell line 3A9 was cloned. Briefly, the heavy and light chain variable region gene-specific cDNAs were synthesized using the SMARTer 5 'RACE Synthesis Kit (TAKARA, # 634859) according to the instructions for use. The 5 'and 3' terminal of the cDNA sequence were modified with the PCR primers and the said primers were designed to have the appropriate leader sequences added to the heavy and light chain variable region cDNAs, respectively, so that the resulting PCR products could be seamlessly cloned into the heavy chain expression vector pHB-Fc and light chain expression vector pHB-Cκ expressed by the existing recombinant antigen-binding proteins. The pHB-Fc expression vector contains the human IgG1 heavy chain constant region gene sequence, wherein the CH2 had the L234A and L235A (Eu numbering) mutations with antigen-binding protein ADCC knockout (KO) effect, and the pHB-Cx vector contains the human κ light chain constant region gene sequence. The PCR amplification products of the heavy and light chain variable regions were cloned into expression vectors using the In-fusion cloning reagent (TAKARA, # 639650) and transformed into E.coli DH5α competent cells (Yeastern Biotech Co., Ltd., #FYE607-80VL). Monoclonal colonies were selected for Sanger and NGS sequencing, and the variable region sequences of antigen-binding proteins were obtained after analysis. The variable region sequence of anti-CD73 antigen-binding protein expressed by 3A9 was as follows:

| | |
|---|---|
| 3A9 VH | SEQ ID No.: 29 |
| EVQLVESGGGLVKPGGSLKLSCAASGFTFSKYAMSWVRQSPEKRLEWVAEISSGGGY INYPDTVTGRFTISRDNAKNTLYLEMSSLRSGDTAMYYCARAIYYYGSSYNYYAMDY WGQGTSVTVSS | |
| 3A9 VL | SEQ ID No.: 30 |
| DIVMTQSHKFMSTSVGDRVSITCKASQDVGTAEAWYQQKPGQSPKLLIYWASTRHTG | |
| | VPDRFTGSGSGTDFTLTIGNVQSEDLADYFCQQYSSYPLTFGGGTKLEIK |

Where the underlined were the CDRs (as defined by IMGT, and all the CDR lists are as follows):

**Table2. CDR Sequence of Murine CD73 Antigen Binding Protein**

| Domain | | Sequences | SEQ ID No. |
|---|---|---|---|
| VH | CDR1 | GFTFSKYA | 1 |
| | CDR2 | ISSGGGYI | 2 |
| | CDR3 | ARAIYYYGS SYNYYAMDY | 3 |
| VL | CDR1 | QDVGTA | 4 |
| | CDR2 | WAS | 5 |
| | CDR3 | QQYSSYPLT | 6 |

### 2.2 Expression of Antigen Binding Proteins

The expression vector obtained in Example 2.1 was amplified in Escherichia coli, and a sufficient amount of plasmids were prepared using an endotoxin-free plasmid extraction kit (# DP117, Tiangen Biotech (Beijing) Co., Ltd.) for transient transfection to express the antigen-binding protein. The host cells used for expression were CHO-S cells (Thermo Fisher (Scientific), # R80007). Each of the two prepared heavy chain vector was mixed with the light chain vector together with polyetherimide (PEI, Polysciences, # 24765-1) to form the liposome complexes, which were used to transfect the CHO-S cells and the cells were incubated in an incubator for 5-7 days. The cell culture supernatant was collected by centrifugation and purified by Protein A affinity chromatography column to obtain the human-mouse antigen-binding protein (No. of 3A9 expressed antigen-binding protein: 900581).

### 2.3 Preparation of Antigen Binding Proteins 900694-900698

The 3D modeling method was used for the humanization of antigen-binding proteins: firstly, 3D structure modeling was performed for murine antigen-binding proteins to select the optimal structure model. Specifically, Discovery Studio and Schrödinger Antibody Modeling methods were used, respectively, and the homology modeling method was applied to select 5-10 optimal structure solutions. The homology modeling method was generally used for modeling of the Loop region. If the alignment results of the CDR amino acid sequence showed that the identity was less than 50%, then the de novo modeling method would be used to build the CDR3 structure model. PDB BLAST was utilized to obtain the 10 antibody crystal structure models with the closest sequence (with a structural resolution higher than 2.5 angstroms), and the optimal structural model was selected by comparing with the models created by automatic modeling. The sequences of the antigen-binding protein variable regions were then compared against the sequences available in the NCBI IgBlast database, and the humanized framework regions (FR regions) on which it would be suitable for constructing the CDR antibody grafted heavy and light chains were finally determined based on identification and analysis.

During the engineering process, based on the conserved amino acid residues in the FR region of the human antibody and the important amino acid residues in the FR region of the antibody, the engineering sites were designed; and the humanized mutation design was performed for the variable regions of the heavy chain and the light chain of the antigen-binding protein 900581 of the present application. And the designed humanized sequence should meet the following requirements: not affecting the structural stability of the antibody, not affecting the binding of antigen-binding protein to the antigen, not introducing the sites of protein modifications such as glycosylation and phosphorylation, and not introducing the sites that are prone to be oxidized and aminated, and enhancing the structural stability. Based on the analysis, three humanized heavy chain sequences and three humanized light chain sequences were designed against the murine antigen-binding protein sequence of 900581; and the plasmids expressing the antigen-binding proteins at the humanized point mutation were expressed in CHO-S cells, respectively, and the humanized antigen-binding proteins were obtained after purification. The receptor binding ability, functional inhibitory activity, non-specific binding properties and thermal stability of the humanized antigen-binding proteins were screened by using the methods of ELISA, Biacore and flow cytometry; and five humanized anti-CD73 antigen-binding proteins with excellent performance were obtained. The protein numbers of the obtained humanized anti-CD73 antigen-binding protein and the corresponding VH and VL sequences are as follows:

| | |
|---|---|
| **900694** VH | SEQ ID No.: 31 |
| EVQLVESGGGLVKPGGSLRLSCAASGFTFSKYAMSWVRQAPGKGLEWVAEISSGGG YINYPDTVTGRFTISRDNAKNSLYLQMNSLRAEDTAVYYCARAIYYYGSSYNYYAMD YWGQGTTVTVSS | |
| **900694** VL | SEQ ID No: 32 |
| DIQMTQSPSSLSASVGDRVTITCRASQDVGTAEAWYQQKPGKAPKLLIYWASTRHTG VPDRFSGSGSGTDFTLTISSLQPEDFATYYCQQYSSYPLTFGQGTKLEIK | |
| **900695 and 900697** | VH SEQ ID No.: 33 |
| QVQLVESGGGLVKPGGSLRLSCAASGFTFSKYAMSWVRQAPGKGLEWVAEISSGGG YINYADSVTGRFTISRDNAKNSLYLQMNSLRAEDTAVYYCARAIYYYGSSYNYYAMD YWGQGTTVTVSS | |
| **900695 and 900698** | VL SEQ ID No.: 34 |
| DIQMTQSPSSLSASVGDRVTITCRASQDVGTAEAWYQQKPGKAPKLLIYWASTRHTG VPSRFSGSGSGTDFTLTISSLQPEDFATYYCQQYSSYPLTFGQGTKLEIK | |
| **900696 and 900698** | VH SEQ ID No.: 35 |
| QVQLVESGGGLVKPGGSLRLSCAASGFTFSKYAMSWIRQAPGKGLEWVAEISSGGGY INYADSVKGRFTISRDNAKNSLYLQMNSLRAEDTAVYYCARAIYYYGSSYNYYAMD YWGQGTTVTVSS | |
| **900696 and 900697** VL | SEQ ID No.: 36 |
| DIQMTQSPSSLSASVGDRVTITCRASQDVGTAEAWYQQKPGKAPKLLIYWASTLQSG VPSRFSGSGSGTDFTLTISSLQPEDFATYYCQQYSSYPLTFGQGTKLEIK | |

Wherein, the underlined regions indicate the CDRs (based on IMGT's definition), and 900694, 900695, 900696, 900697, and 900698 are the numbers of the 5 humanized anti-CD73 antigen-binding proteins, respectively.

### Example 3 Detection of Binding Activity of Anti-CD73 Antigen Binding Protein to Human CD73 on Cell Surface

Detection of the binding activity of humanized antigen-binding proteins 900694, 900695, 900696, 900697, and 900698 to CD73 antigen-binding proteins 900581, 900565, and 900581 to human CD73 on the cell surface (CHOK1-huCD73-3F4, Huabo Biopharm). Wherein, the chimeric antibody 900565 was derived from hybridoma cell line 115B10. An amino acid sequence of the heavy chain variable region (VH) of 900565 is set forth in SEQ ID No.: 41 and an amino acid sequence of the light chain variable region (VL) of 900565 is set forth in SEQ ID No.: 42.

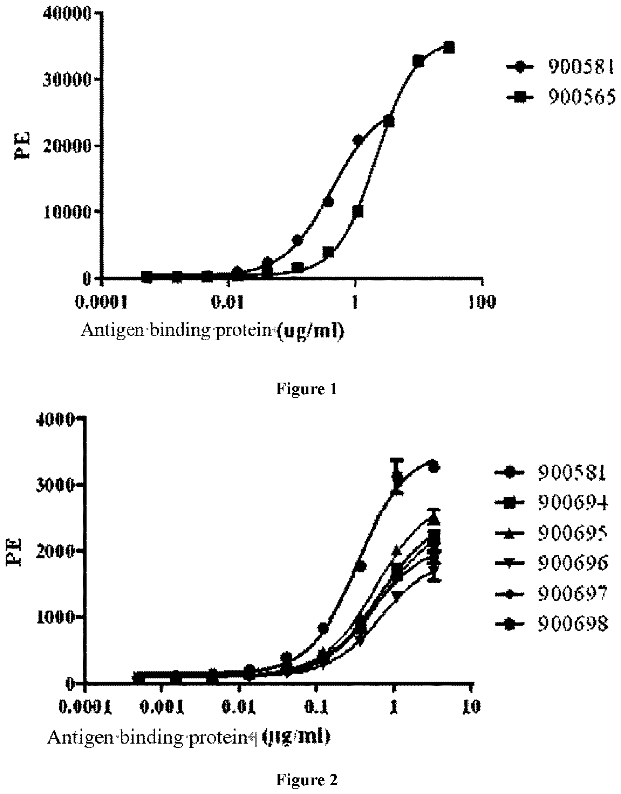

All the antigen-binding proteins were diluted to 30 µg/ml with PBS solution containing 1% BSA (1% BSA/PBS), diluted at 3-fold into 11 gradients and then added to a 96-well U-shaped plate at 20 µL/well, and negative control (1% BSA/PBS was added only) was set up simultaneously. The suspension of human CD73-expressing cells (CHOK1-huCD73-3F4, Huabo Biopharm) in the logarithmic growth phase were collected and centrifuged at 1000 rpm for 5 min, and the culture medium was discarded; then the cells were resuspended with 1% BSA/PBS to a viable cell density of 1 × 10⁶/mL, and then added into the 96-well U-shaped plate already added with anti-CD73 antigen-binding protein at 20 µL (2×10⁴ cells)/well for reaction at room temperature for 30 min. After the reaction, the cells in the 96-well U-shaped plate were resuspended with 1% BSA/PBS and centrifuged at 300 g for 3 min and the supernatant solution was discarded; after washing once in this manner, the cells were added with PE-goat anti-human-Fc (Jackson ImmunoResearch, # 109-115-098) diluted at 1:200 fold, and allowed for reaction at room temperature and protected from light for 15 min. After the reaction, the cells in the 96-well U-shaped plate were resuspended with 1% BSA/PBS and centrifuged at 300 g for 3 min and the supernatant was discarded; after washing in this manner 3 times, and the cells were finally resuspended with 1% BSA/PBS at 100 µL/well, and the fluorescence intensity of the PE channel was measured with a flow cytometer (BD, # Canto II). The detection results of the binding activity of 900581 antigen-binding protein are shown in Figure 1 and Table 3, and those of 900694 to 900698 antigen-binding protein are shown in Figure 2 and Table 4. These results indicated that 900581 antigen-binding protein had a good affinity to cells expressing human CD73 on the cell surface (CHOK1-huCD73-3F4, Huabo Biopharm), and the binding activities of 900694 to 900698 were comparable to that of 900581.

**Table3. Binding Activity of 900581 and 900565 to Human CD73 on Cell Surface**

| Antigen binding protein | 900581 | 900565 |
|---|---|---|
| EC50(µg/mL) | 0.4258 | 2.141 |

**Table4. Binding Activity of 900581 and 900694 to 900698 to Human CD73 on Cell Surface**

| Antigen binding protein | 900581 | 900694 | 900695 | 900696 | 900697 | 900698 |
|---|---|---|---|---|---|---|
| EC50(µg/mL) | 0.3466 | 0.6461 | 0.5942 | 0.6840 | 0.5176 | 0.6879 |

### Example 4 Detection of Inhibitory Effect of Anti-CD73 Antigen Binding Protein on Enzymatic Activity of Recombinant CD73

Detection of the inhibitory effects of 900581 and 900694, 900695, 900696, 900697 and 900698 on the protease activity of recombinant CD73 protein (ACRO, # CD3-H52H7-100 µg). The specific operation steps are as follows: a series of CD73 antigen-binding proteins were diluted to 120 µg/mL with TM Buffer (ROCKLAND, # MB-059), and then diluted at 5-fold into 10 gradients, and then added at 25 µL/well into a 96-well plate. The recombinant CD73 proteins were then diluted to 0.3 µg/ml with TM Buffer (ROCKLAND, # MB-059), and each of the antigen-binding proteins was added at 25 µL/well to a 96-well plate, and mixed well. Then the diluted AMP and ATP were mixed in a ratio of 1:1, and added to the above 96-well plate at 50 µL/well and allowed for the reaction for 15 min. Finally, the CellTiter-Glo^{®} substrate was added into the CellTiter-Glo^{®} buffer, mixed well and equilibrated to room temperature, then added into the test well at 100 µL/well, and allowed for reaction at room temperature for 10 min, and then detected at full wavelength.

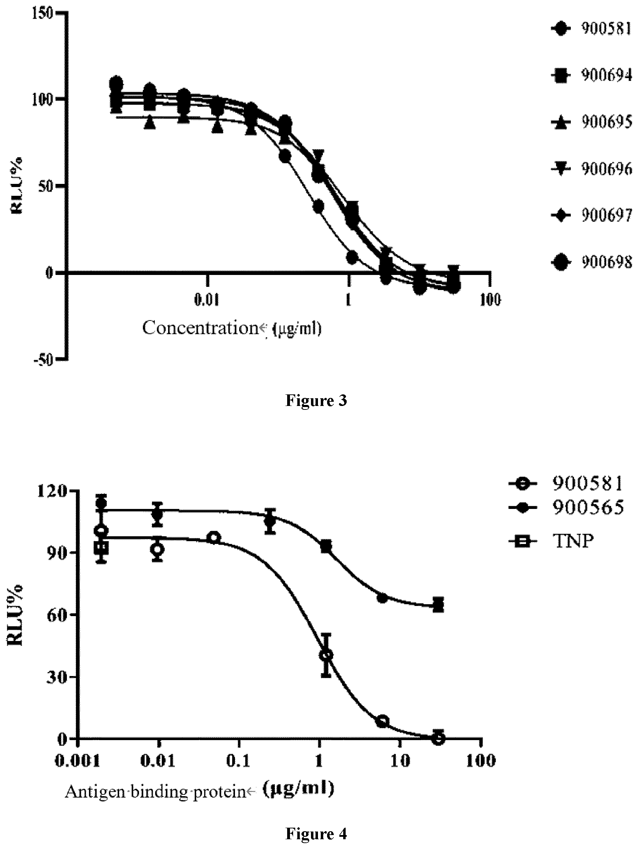

The detection results of the inhibitory effects of 900581, 900694, 900695, 900696, 900697 and 900698 on the enzymatic activity of recombinant CD73 protein (ACRO, # CD3-H52H7-100 µg) are shown in Figure 3 and Table 5. The results showed that the inhibitory effects of 900694, 900695, 900696, 900697 and 900698 on the protease activity of recombinant CD73 were comparable to that of 900581.

**Table5. Inhibitory Effect of 900581 and 900694 to 900698 on Protease Activity of Recombinant CD73**

| Antigen binding protein | 900581 | 900694 | 900695 | 900696 | 900697 | 900698 |
|---|---|---|---|---|---|---|
| EC50(µg/mL) | 0.2621 | 0.6812 | 0.7460 | 0.7509 | 0.5854 | 0.5876 |

### Example 5 Detection of Inhibitory Effect of Anti-CD73 Antigen Binding Protein on Enzymatic Activity of CD73 on Cell Surface

All the antigen-binding proteins were diluted to 30 µg/ml in PBS solution and diluted at 5-fold into 6 gradients. The suspension of human CD73-expressing cells (CHOK1-huCD73-3F4, Huabo Biopharm) in the logarithmic growth phase was prepared into 4×10⁵/ml cell suspension with culture medium and added into a 96-well U-shaped plate at 100ul/well (about 40,000 cells), and centrifuged at 1500 rpm for 5 min and then the supernatant was discarded. Then, the cells were resuspended with serially diluted antigen-binding protein, added into each well at 100 uL/well, mixed well, and incubated at 37°C for 20 min. After the end of the incubation, the cells were centrifuged at 1500 rpm for 5 min, the supernatant was discarded, and washed once with PBS at 200 ul/well, and then resuspend with 180 µM AMP at 100 µL/well, and incubated at 37°C for 60 min. After the end of the incubation, the cells were centrifuged at 1500 rpm for 5 min, and the supernatant was pipetted at 50 µL/well to a 96-well black plate, and added with 50 µL/well prepared ATP, mixed well, and reacted at 37°C for 15 min. Finally, the CellTiter-Glo^{®} substrate was added into the CellTiter-Glo^{®} buffer, mixed well and equilibrated to room temperature, then added into the test well at 100 µL/well, and allowed for reaction at room temperature for 10 min, and then detected at full wavelength. The percentage of residual enzyme activity was assessed as follows: residual activity of CD73 was: (CHOK1-huCD73-3F4 cells + Ab + ATP + AMP)- (ATP + AMP)/(CHOK1-huCD73-3F4 cells + ATP + AMP)- (ATP + AMP) * 100.

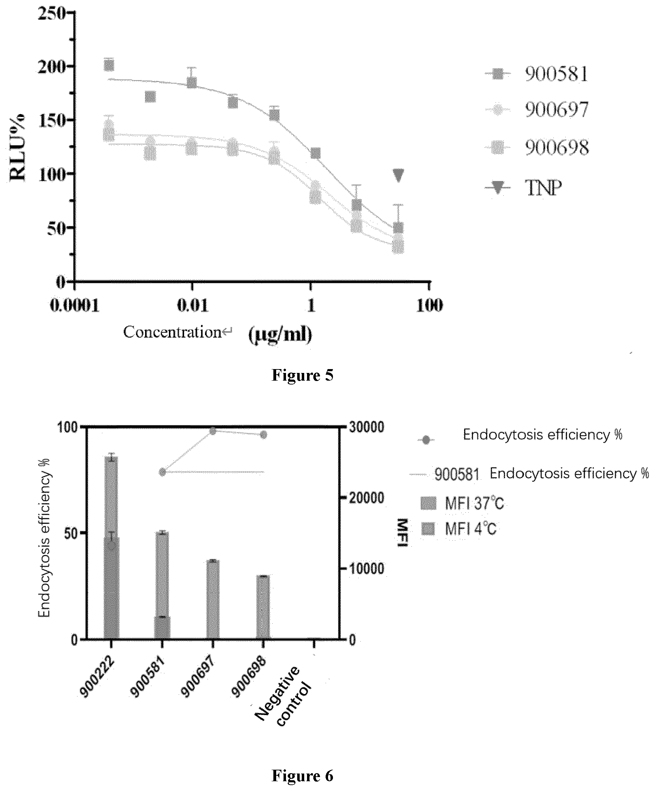

The inhibitory effects of anti-CD73 chimeric antibodies 900581 and 900565 on the enzymatic activity of CD73 cells (CHOK1-huCD73-3F4, Huabo Biopharm) are shown in Figure 4 and Table 6; and the inhibitory effects of 900581, 900697 and 900698 on the enzymatic activity of CD73 cells (CHOK1-huCD73-3F4, Huabo Biopharm) are shown in Figure 5 and Table 7. These results indicated that the inhibitory effects of 900697, 900698 and 900581 on the enzymatic activity of CD73 on cell membrane were basically equivalent.

**Table6. Inhibitory Effects of 900581 and 900565 on Enzyme Activity of CD73 on Cell Membrane**

| Antigen binding protein | 900581 | 900565 |
|---|---|---|
| EC50(µg/mL) | 0.9437 | 1.625 |

**Table7. Inhibitory Effects of 900581, 900697 and 900698 on the Enzyme Activity of CD73 on Cell Membrane**

| Antigen binding protein | 900581 | 900697 | 900698 |
|---|---|---|---|
| EC50(µg/mL) | 1.853 | 1.972 | 1.431 |

### Example 6 Detection of Binding Affinity of Anti-CD73 Antigen Binding Protein to Human CD73

The binding kinetics of 900581, 900694, 900695, 900696, 900697 and 900698 to human CD73 protein (manufacturer: ACRO Biosystems, Cat No.: CD3-H52H7) was determined using Biacore (GE, model: T200), HBS-EP + buffer (pH 7.4), and protein A chip (manufacturer: GE, Cat No.: 29-1275-56).

The temperatures of the sample chamber and flow path were set at 25°C. The protein A chips separately captured the antigen-binding proteins as ligands, which were immobilized at about 100 RU. Human CD73 proteins were diluted to a series of concentrations of 50 nM, 25 nM, 12.5 nM, 6.25 nM, 3.12 nM, and 1.57 nM with the experimental buffer and used as the analytes; and the experimental buffer solution was taken as the control at the concentration of 0, and the assay was conducted at the flow rate of 30 µL /min, with binding for 120 s and dissociation for 600 s. Ten mM Glycine, pH 1.5, was regenerated for 60s at a flow rate of 50 µL/min for multiple cycle kinetic detection. The binding rate constant (ka), dissociation rate constant (kd) and dissociation equilibrium rate constant (KD) were fitted using the Fit local mode of a 1:1 binding model. The fitting results are shown in Table 8, which indicated that all the antigen-binding proteins could bind to human CD73 protein, and there was no significant difference in the binding affinity to human CD73 protein between most of these antigen-binding proteins.

**Table 8: Detection of Binding Affinity of 900581 and 900694 to 900698 to Human CD73 Protein**

| Ligand | Analyte | ka (1/Ms) | kd (1/s) | KD (M) |
|---|---|---|---|---|
| 900581 | Human CD73 | 2.67E+05 | 4.39E-04 | 1.65E-9 |
| 900694 | Human CD73 | 3.29E+05 | 4.93E-04 | 1.50E-9 |
| 900695 | Human CD73 | 2.95E+04 | 6.03E-05 | 2.04E-9 |
| 900696 | Human CD73 | 2.19E+05 | 9.28E-04 | 4.23E-9 |
| 900697 | Human CD73 | 3.80E+05 | 4.92E-04 | 1.29E-9 |
| 900698 | Human CD73 | 3.39E+05 | 1.06E-03 | 3.12E-9 |

### Example 7 Detection of Binding Affinity of Anti-CD73 Antigen Binding Protein to CD73 of Different Species (Human, Mouse, Rat and Monkey)

The binding kinetics of 900581, 900694, 900695, 900696, 900697 and 900698 to human CD73 protein (manufacturer: ACRO Biosystems, Cat No.:CD3-H52H7), mouse CD73/NT5E (manufacturer: ACRO Biosystems, Cat No.:CD3-M52H9), rat CD73 (manufacturer: Novoprotein, Cat No.: CB16) and monkey CD73 (manufacturer: Novoprotein, article number: CI21) were separately determined using the Biacore (GE, model: T200) and protein A chip (manufacturer: GE, Cat No.: 29-1275-56) and HBS-EP + (pH 7.4) as the experimental buffer. The temperatures of the sample chamber and flow path were set at 25°C. The protein A chips separately captured the antigen-binding proteins as ligands, which were immobilized at about 100 RU. Human CD73 proteins/monkey CD73/rat CD73/mouse CD73 were separately diluted to a series of concentrations of 50 nM, 25 nM, 12.5 nM, 6.25 nM, 3.12 nM, and 1.57 nM with the experimental buffer and used as the analytes; and the experimental buffer solution was taken as the control at the concentration of 0, and the assay was conducted at the flow rate of 30 µL /min, with binding for 120 s and dissociation for 600 s. Ten mM Glycine, pH 1.5, was regenerated for 60s at a flow rate of 50 µL/min for multiple cycle kinetic detection. The binding rate constant (ka), dissociation rate constant (kd) and dissociation equilibrium rate constant (KD) were fitted using the Fit local mode of a 1:1 binding model. The fitting results are shown in Table 9, which indicated that all the aforesaid antigen-binding proteins could bind to human CD73 and monkey CD73 (manufacturer: Novoprotein, Cat No.: CI21), and there was no significant difference in the binding affinity to human CD73 and monkey CD73 proteins between most of these antigen-binding proteins. Meanwhile, all the antigen-binding proteins did not bind to mouse (manufacturer: ACRO Biosystems, Cat No.: CD3-M52H9) and rat (manufacturer: Novoprotein, Cat No.: CB16) CD73 proteins.

**Table 9: Detection of Binding Affinity of 900581 and 900694~900698 to Monkey CD73 Protein**

| Ligand | Analyte | ka (1/Ms) | kd (1/s) | KD (M) |
|---|---|---|---|---|
| 900581 | Monkey CD73 | 3.49E+05 | 3.58E-04 | 1.05E-9 |
| 900694 | Monkey CD73 | 5.78E+05 | 1.17E-04 | 2.03E-9 |
| 900695 | Monkey CD73 | 1.80E+05 | 1.48E-04 | 8.22E-10 |
| 900696 | Monkey CD73 | 3.54E+05 | 2.14E-04 | 6.04E-10 |
| 900697 | Monkey CD73 | 6.41E+04 | 1.37E-04 | 2.13E-9 |
| 900698 | Monkey CD73 | 1.08E+05 | 1.15E-04 | 1.41E-9 |

### Example 8 Detection of the Ability of Anti-CD73 Antigen Binding Protein to Induce Endocytosis of CD73 on Cell Surface

Control protein 900222, chimeric protein 900581 and humanized proteins 900697 and 900698 were diluted to 1 µg/ml in PBS solution in duplicate wells for each concentration. Among them, the sequence of control protein 900222 was derived from the mAb-CD73.4-IgG2 antibody sequence in the patented CD73 antibody (US20190284293A1) of Bristol-Myers Squibb. Calu6 cells in the logarithmic growth phase were prepared into cell suspension at 2×10⁵/ml and added into a 96-well U-shaped plate at 100 µL/well (about 20,000 cells) was centrifuged at 300 g for 3 min, and the supernatant was discarded. Then, the cells were resuspended with diluted antigen-binding proteins, added into each well at 100 uL/well, mixed well, and incubated at 4°C for 30 min. After the end of the incubation, the suspension was centrifuged at 300g for 3 min, the supernatant was discarded, the cells were resuspended with 1% BSA, and half of the cells were incubated at 37°C, and the other half at 4°C for 5 h. After the end of the incubation, the wells at the corresponding time were removed, and centrifuged at 300 g for 3 min; after the supernatant was discarded, PE anti-huIgG Fc was added (1:200) at 50 µL/well, mixed well, and reacted at 4°C for 30 min. After the end of the incubation, the cells were washed twice and centrifuged at 300 g for 3 min, and the fluorescence intensity was detected by flow cytometry. The detection results of anti-CD73 antigen-binding protein induced endocytosis of CD73 on the cell surface are shown in Table 10 and Figure 6 below. The results indicated that the effect of chimeric protein 900581 and humanized proteins 900697 and 900698 in inducing endocytosis of CD73 was significantly superior than that of control protein 900222, and the effect of humanized proteins 900697 and 900698 in inducing endocytosis was superior than that of chimera 900581.

**Table10. Efficiency of Anti-CD73 Antigen Binding Protein in Inducing CD73 Endocytosis**

| Antigen binding protein | 900222 | 900581 | 900697 | 900698 |
|---|---|---|---|---|
| Endocytosis efficiency (%) | 44.18 | 78.75 | 98.17 | 96.35 |

### Example 9 Identification of Binding Epitopes of Anti-CD73 Antigen Binding Proteins

### 9.1 Detection of Binding Epitopes of 900581 Antigen Binding Protein by Hydrogen Deuterium Exchange Mass Spectrometry (HDX-MS)

Five to 10 µM antigen, antigen-binding protein or antigen-binding protein complex (1: 1 molar ratio) (50 mM HEPES, pH 7.4, 150 mM NaCl, 4 mM TCEP) were allowed to stand at 4°C for 1 h, respectively until it reached the stable state of a complex. Then 5 µL of the sample was subsequently diluted into 20 µL of D₂O (deuterium) at 4°C and allowed to stand for different HDX time points (e.g., 0, 10, 60, 300, 900 seconds) until mass spectrometry. After a period of hydrogen-deuterium exchange, the reaction was terminated by mixing the solution with 25 µL of ice-cold 4 M guanidine hydrochloride and 1% trifluoroacetic acid. Immediately after the termination of the reaction, the sample tube was placed on dry ice until the sample was injected into the LEAP-HDX-PAL 3.0 platform. After being injected into the fully automated hydrogen-deuterium exchange platform, the sample flew through the immobilized pepsin column at a flow rate of 120 µL/min, and the enzymatically cleaved peptide fragments were captured on a C18 capture column and desalted. Desalted peptide fragments were separated with a 2.1 mm × 5 cm C18 column (1.9 µm Hypersil Gold, Thermo Fisher) with a linear gradient of 4% to 40% acetonitrile in 0.3% formic acid within 8 min. During sample processing, enzymatical cleavage of protein and peptide fragment separation was performed at 4°C. The hydrogen-deuterium exchange mass spectrometry data were obtained using an Orbitrap mass spectrometer (Orbitrap Fusion^{™} Tribrid^{™} Mass Spectrometer, Thermo Fisher) and the measured resolution was 65,000 (m/z 400). There were three HDX measurements (triplicates) for each sample at each time point.

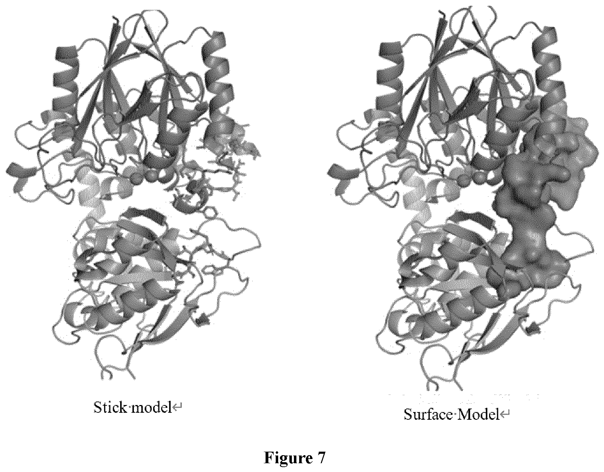

The results of hydrogen-deuterium exchange mass spectrometry (HDX-MS) demonstrated that the binding epitopes of 900581 mainly included 143-NIKAKGPLASQISGL-157 region (SEQ ID No.: 37) and 178-SKETPFLSNPGTNL-191 region (SEQ ID No.: 38) of the N-terminal domain of CD73, and 381-WNHVSM-386 region (SEQ ID No.: 39) of the C-terminal domain of CD73. After these three non-contiguous regions were mapped to the three-dimensional structure model of CD73, this epitope was found to be at the edge of the catalytic active center of CD73, as set forth in Figure 7. When 900581 binds to this epitope, it could block the catalytic center of CD73 in a very efficient manner, thus inhibiting the enzymatic activity of CD73 with maximum efficiency. The retrieval of the patents and literature reports showed that 900581 developed by our company is so far the first reported therapeutic antigen-binding protein which binds around the catalytic center of CD73 and for which the epitope information has been provided.

### 9.2 Validation of Binding Epitopes of Antigen-binding Protein 900581 by Competitive Binding Assay with APCP, a Non-Hydrolyzed AMP Analogue

Conformational change from open to closed conformation occurred after CD73 bound to its substrate AMP. Substitution of AMP with APCP, the non-hydrolyzed AMP analog could lock CD73 in the catalytic intermediate state in the closed conformation as APCP could not hydrolyze to generate adenosine. With the increase in the concentration of APCP, the fraction of CD73 in the closed catalytic intermediate state increased, and the fraction of CD73 bound to antigen-binding proteins in the catalytic region decreased, whereas the fraction of CD73 bound to antigen-binding proteins in the noncatalytic region had no change. The specific operating steps of the method were as follows: the cells were prepared into 1×10⁶/ml cell suspension with 1% BSA, and the mixed solution was added to a 96-well U-shaped plate at 20 µL/well. The APCP was diluted into 30 nM/30 µM, respectively with 1% BSA, and added to the above 96-well U-shaped plate at 20 µL/well, mixed well, and allowed to stand at room temperature for 30 min. The test antigen-binding proteins were diluted to 90 µg/ml, and then diluted at 3-fold into 10 gradients, respectively. The serially diluted antigen-binding proteins were added to the above 96-well U-shaped plate at 20 µL/well, mixed well, and allowed to stand at room temperature for 30 min. At the end of incubation, the proteins were centrifuged at 300 g for 3 min, and after the supernatant was discarded, the proteins were added with the secondary antibody PE goat anti-human IgG Fc (1:200) at 20 µL/well, mixed well, and allowed to stand at room temperature for 15 min. After the end of the incubation, the proteins were washed twice and centrifuged at 300 g for 3 min and the PE-MFI was detected by flow cytometry.

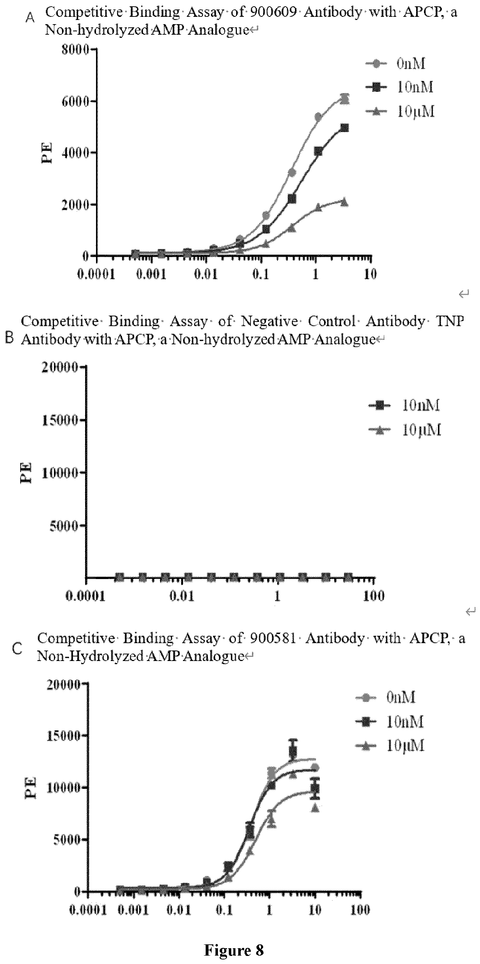

The results are shown in Figures. 8A-8C, where 900609 is the CPI-006 antibody provided by Corvus, which had been shown to bind around the catalytic center, and TNP is the negative control antibody. The 900581 antigen-binding protein behaved similarly to the CPI-006 antibody: with a gradual decrease in the fraction of antigen-binding protein bound to CD73 as the concentration of APCP increased, which further demonstrated that 900581 bound around the catalytic active center of CD73.

### Example 10 Detection of in Vivo Therapeutic Effect of Anti-CD73 Antigen Binding Protein on Tumors

10.1
In this experiment, the CD73 humanized mice were used to construct the MDA-MB-231 triple-negative breast cancer animal model and the therapeutic efficacy of the test antibody was tested. First, 100 µL of MDA-MB-231 (2×10⁶ cells) from ATCC cell bank were inoculated into CD73 humanized mice to construct the MDA-MB-231 triple-negative breast cancer animal model in CD73 humanized mice. Tumor-forming mice were equally divided into 4 experimental groups according to tumor volume and body weight, with 6 mice in each group, and all the animals were intraperitoneally injected with the corresponding drugs twice a week for a total of 6 times. The specific dosing regimens are outlined in the table below. Wherein, 900201 was the negative control antibody that did not bind to CD73 antigen. An amino acid sequence of the heavy chain (HC) of 900201 is set forth in SEQ ID No.: 43 and that of the light chain (LC) of 900201 is set forth in SEQ ID No.: 44.

The results showed that 900581, 900697, and 900698 could significantly inhibit the growth of MDA-MB-231 triple-negative breast cancer cells, and had no impact on the body weight of the mice. Whereas the negative control antibody 900201 could not inhibit the tumor growth.

**Table 11 Dosing Regimen**

| Group | Test article | Number of animals | Route of administration | Dosing frequency^{a} | Dosing times |
|---|---|---|---|---|---|
| G1 | 900201 | 6 | i.p. | BIW | 6 |
| G2 | 900581 | 6 | i.p. | BIW | 6 |
| G3 | 900697 | 6 | i.p. | BIW | 6 |
| G4 | 900698 | 6 | i.p. | BIW | 6 |

10.2
The NPG mice were used to construct the MDA-MB-231 triple negative breast cancer animal model and the therapeutic efficacy of the test antibody was tested. The MDA-MB-231 human breast cancer cells used in this study were cultured in L-15 medium supplemented with 10% FBS in a CO₂ -free incubator at 37°C. Prior to 10 consecutive subcultures, approximately 5.0×10⁶ MDA-MB-231 cells were suspended in 100 µL PBS and mixed with an equal volume of Matrigel and then inoculated subcutaneously into the right side of the back near the axilla of NPG humanized mice with a volume of approximately 200 µL. Mice were anesthetized with 2% to 5% isoflurane prior to inoculation. On the day of inoculation, 1.0×10⁷ PBMCs (100 µL) were injected into the mice via the tail vein, and when the tumors grew to an average volume of approximately 50-80 mm³, 18 tumor-bearing mice were randomly divided into 3 groups with 6 mice in each group based on tumor volume and body weight. Dosing was performed on the day of grouping. See Table 12 for the specific dosing regimen. Of these, 900201 was a negative control antibody that could not bind to CD73 antigen. An amino acid sequence of the heavy chain (HC) of 900201 is set forth in SEQ ID No.: 43 and that of the light chain (LC) of 900201 is set forth in SEQ ID No.: 44. Olecumab is an anti-CD73 monoclonal antibody developed by AstraZeneca.

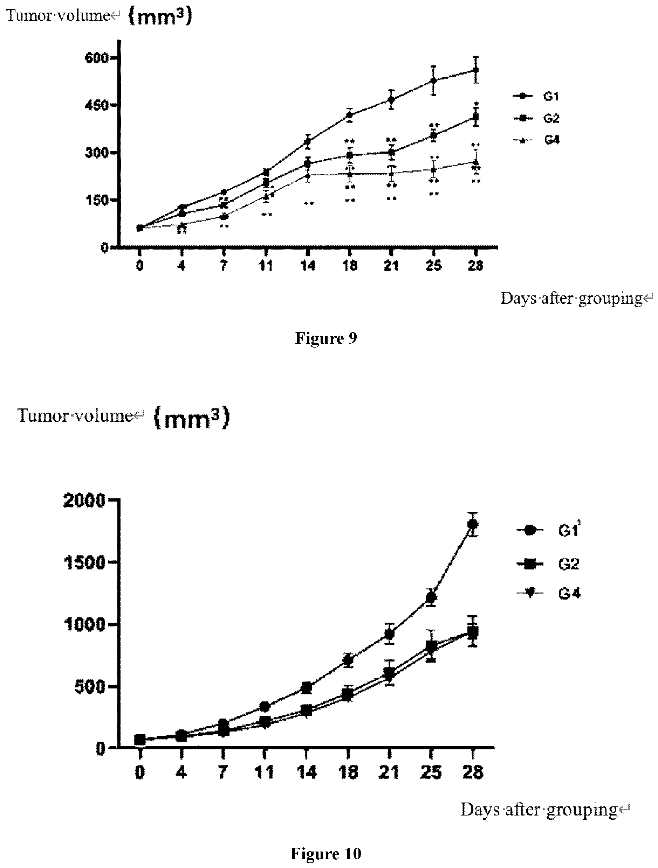

As set forth in Table 12 and Figure 9 below, oleclumab and 900698 could significantly inhibit the growth of MDA-MB-231 triple-negative breast cancer cells, whereas the negative control antibody 900201 failed to inhibit tumor growth.

**Table 12 Dosing Regimen for Triple Negative Breast Cancer Model**

| Group | Test article | Number of animals | Dose administered (mg/kg) | Route of administration | Dosing frequency^{a} | Dosing times |
|---|---|---|---|---|---|---|
| G1 | 900201 | 6 | 5 | i.p. | BIW | 8 |
| G2 | oleclumab | 6 | 5 | i.p. | BIW | 8 |
| G4 | 900698 | 6 | 5 | i.p. | BIW | 8 |

### Example 11 Detection of in Vivo Therapeutic Effect of Anti-CD73 Antigen Binding Protein on Pancreatic Cancer

In this study, NPG mice were used to construct the animal model of BxPC-3 pancreatic cancer and evaluate the therapeutic efficacy of the test antibody. The human pancreatic cancer cell line BxPC-3 used in this study was cultured in RPMI-1640 medium supplemented with 10% FBS in a 5% CO₂ incubator at 37°C. Prior to 10 continuous subcultures of the cells, 100 µL of PBS containing approximately 1×10⁷ BxPC-3 cells and an equal volume of Matrigel were mixed well and inoculated subcutaneously into the right side of the back near the axilla of 18 NPG mice with an inoculation volume of 200 µL. Mice were anesthetized with 2% to 5% isoflurane prior to inoculation. On the day of inoculation, 1.0×10⁷ PBMCs (100 µL) were injected into the mice via the tail vein, and when the tumors grew to an average volume of approximately 50-80 mm³, 18 tumor-bearing mice were randomly divided into 3 groups with 6 mice in each group based on tumor volume and body weight. Dosing was performed on the day of grouping. See Table 13 for the specific dosing regimen. Of these, 900543 was a negative control antibody (900543 was the ADCC knock-out version of 900201) that could not bind to CD73 antigen. An amino acid sequence of the heavy chain (HC) of 900543 is set forth in SEQ ID No.: 47 and that of the light chain (LC) of 900543 is set forth in SEQ ID No.: 44. Olecumab is an anti-CD73 monoclonal antibody developed by AstraZeneca.

As shown in Table 13 and Figure 10 below, oleculumab and 900698 significantly inhibited the growth of BxPC-3 pancreatic cancer cells, whereas the negative control antibody 900543 failed to inhibit tumor growth.

**Table 13 Dosing Regimen for Pancreatic Cancer Model**

| Group | Test article | Number of animals | Dose administered (mg/kg) | Route of administration | Dosing frequency^{a} | Dosing times |
|---|---|---|---|---|---|---|
| G1' | 900543 | 6 | 5 | i.p. | BIW | 8 |
| G2 | oleclumab | 6 | 5 | i.p. | BIW | 8 |
| G4 | 900698 | 6 | 5 | i.p. | BIW | 8 |

All the literature references referred to in the present application are cited as references in the present application, just as each document is cited separately as a reference. It will also be understood that, after reading the above contents of the present application, the contents of the present application may be changed or modified by those skilled in the art, such equivalent forms also fall into the scope limited by the claims annexed to the present application.

## Claims

1. An isolated antigen-binding protein comprises HCDR3, wherein said HCDR3 comprises an amino acid sequence as set forth in SEQ ID No.: 3.

2. The isolated antigen-binding protein of claim 1 comprises HCDR2, wherein said HCDR2 comprises an amino acid sequence as set forth in SEQ ID No.:2.

3. The isolated antigen-binding protein according to any one of claims 1-2, comprises HCDR1, wherein said HCDR1 comprises an amino acid sequence as set forth SEQ ID No.:1.

4. The isolated antigen-binding protein according to any one of claims 1-3, comprises LCDR3, wherein said LCDR3 comprises an amino acid sequence as set forth SEQ ID No.:6.

5. The isolated antigen-binding protein in any one of claims 1-4, comprises LCDR2, wherein said LCDR2 comprises an amino acid sequence as set forth in SEQ ID No.:5.

6. The isolated antigen-binding protein according to any one of claims 1-5, comprises LCDR1, wherein said LCDR1 comprises an amino acid sequence as set forth in SEQ ID No.:4.

7. The isolated antigen-binding protein according to any one of claims 3-6, comprises H-FR1;wherein the C terminal of said H-FR1 is directly or indirectly linked to the N terminal of the HCDR1, and said H-FR1 comprises an amino acid sequence as set forth in SEQ ID No.:7, SEQ ID No.:15, or SEQ ID No.:19.

8. The isolated antigen-binding protein according to any one of claims 2-7, comprises H-FR2, wherein said H-FR2 is located between the said HCDR1 and said HCDR2, and the said H-FR2 comprises an amino acid sequence as set forth in SEQ ID No.: 8, SEQ ID No.:16, or SEQ ID No.: 21.

9. The isolated antigen-binding protein according to any one of claims 2-8, comprises H-FR3, wherein said H-FR3 is located between said HCDR2 and the said HCDR3, and the said H-FR3 comprises an amino acid sequence as set forth in SEQ ID No.: 9, SEQ ID No.: 17, SEQ ID No.: 20 or SEQ ID No.: 22.

10. The isolated antigen-binding protein according to any one of claims 1-9, comprises H-FR4, wherein the N-terminal of said H-FR4 is linked to the C-terminal of the HCDR3, and said H-FR4 comprises an amino acid sequence as set forth in SEQ ID No.:10 or SEQ ID No.:18.

11. The isolated antigen-binding protein according to any one of claims 6-10 comprises L-FR1, wherein the C-terminal of said L-FR1 is directly or indirectly linked to the N-terminal of the LCDR1, and the said L-FR1 comprises an amino acid sequence as set forth in SEQ ID No.: 11 or SEQ ID No.: 23.

12. The isolated antigen-binding protein according to any one of claims 6-11, comprises L-FR2, wherein said L-FR2 is located between said LCDR1 and said LCDR2, wherein said L-FR2 comprises an amino acid sequence as set forth in SEQ ID No.:12 or SEQ ID No.:24.

13. The isolated antigen-binding protein according to any one of claims 5-12, comprises L-FR3, wherein said L-FR3 located between said LCDR2 and said LCDR3, and said L-FR3 comprises an amino acid sequence as set forth in SEQ ID No.: 13, SEQ ID No.: 25, SEQ ID No.: 27 or SEQ ID No.: 28.

14. The isolated antigen-binding protein according to any one of the claims 4-13, comprises L-FR4, wherein the N-terminal of said L-FR4 is linked to the C-terminal of said LCDR3, and said L-FR4 comprises an amino acid sequence as set forth in SEQ ID No.: 14 or SEQ ID No.: 26.

15. The isolated antigen-binding protein according to any one of the claims 6-14, wherein said HCDR3 comprises an amino acid sequence as set forth in SEQ ID No.: 3; said HCDR2 comprises an amino acid sequence as set forth in SEQ ID No.: 2; said HCDR1 comprises an amino acid sequence as set forth in SEQ ID No.: 1; said LCDR3 comprises an amino acid sequence as set forth in SEQ ID No.: 6; said LCDR2 comprises an amino acid sequence as set forth in SEQ ID No.: 5, and said LCDR1 comprises an amino acid sequence as set forth in SEQ ID No.: 4.

16. The isolated antigen-binding protein according to any one of claims 1-15, comprises VH, wherein said VH comprises an amino acid sequences set forth in SEQ ID No.:29, SEQ ID No.:31, SEQ ID No.:33 or SEQ ID No.:35.

17. The isolated antigen-binding protein according to any one of claims 1-16, comprises VL, wherein said VL comprises an amino acid sequence as set forth in SEQ ID No.: 30, SEQ ID No.:32, SEQ ID No.:34 or SEQ ID No.:36.

18. The isolated antigen-binding protein of claim 17, comprises VH and VL selected from any one of the following groups:
1) said VH comprises an amino acid sequence as set forth in SEQ ID No.: 29 and said VL comprises an amino acid sequence as set forth in SEQ ID No.: 30;
2) said VH comprises an amino acid sequence as set forth in SEQ ID No.: 31 and said VL comprises an amino acid sequence as set forth in SEQ ID No.: 32;
3) said VH comprises an amino acid sequence as set forth in SEQ ID No.: 33 and said VL comprises an amino acid sequence as set forth in SEQ ID No.: 34;
4) said VH comprises an amino acid sequence as set forth in SEQ ID No.: 35 and said VL comprises an amino acid sequence as set forth in SEQ ID No.: 36;
5) said VH comprises an amino acid sequence as set forth in SEQ ID No.: 33 and said VL comprises an amino acid sequence as set forth in SEQ ID No.: 36; and
6) said VH comprises an amino acid sequence as set forth in SEQ ID No.: 35 and said VL comprises an amino acid sequence as set forth in SEQ ID No.: 34.

19. The isolated antigen-binding protein according to any one of claims 1-18, comprises a heavy chain constant region of an antibody.

20. The isolated antigen-binding protein of claim 19, wherein said heavy chain constant region of the antibody is derived from the heavy chain constant region of a human antibody.

21. The isolated antigen-binding protein according to any one of claims 19-20, wherein said heavy chain constant region of an antibody is derived from a human IgG heavy chain constant region.

22. The isolated antigen-binding protein according to any one of claims 19-21, wherein said heavy chain constant region of an antibody is derived from a human IgG1 heavy chain constant region.

23. The isolated antigen-binding protein according to any one of claims 1-22, comprises the light chain constant region of an antibody.

24. The isolated antigen-binding protein of claim 23, wherein said light chain constant region of an antibody is derived from the human Igκ constant region.

25. The isolated antigen-binding protein according to any one of claims 1-24, comprises an antibody or antigen-binding fragment thereof.

26. The isolated antigen-binding protein of claim 25, wherein said antigen-binding fragment comprises a Fab, Fab', Fv fragment, F (ab')₂, F (ab)₂, scFv, di-scFv and/or dAb.

27. The isolated antigen-binding protein according to any one of claims 25-26, wherein said antibody is selected from one or more of the group consisting of: a monoclonal antibody, a chimeric antibody, a humanized antibody, and a fully humanized antibody.

28. The isolated antigen-binding protein according to any one of claims 1-27, which is able to specifically bind to CD73 or a functionally active fragment thereof.

29. The isolated antigen-binding protein of claim 28, said CD73 comprises human CD73 and/or monkey CD73.

30. The isolated antigen-binding protein according to any one of claims 1-29, which is capable of preventing or reducing the activation of CD73.

31. The isolated antigen-binding protein according to any one of claims 28-30, when binding to said CD73, the isolated antigen-binding protein is able to bind to the amino acids at positions 143 to 157 as set forth in SEQ ID No.:40 or at least one of the amino acid residues of a CD73 sequence corresponding thereto, to the amino acids at positions 178 to 191 as set forth in SEQ ID No.:40 or at least one of the amino acid residues of a CD73 sequence corresponding thereto, and to the amino acids at positions 381 to 386 as set forth in SEQ ID No.:40 or at least one of the amino acid residues of a CD73 sequence corresponding thereto.

32. The isolated antigen-binding protein of claim 31, wherein said amino acids at positions 143-157 or a CD73 sequence corresponding thereto are located in the N-terminal domain of CD73; said amino acids at positions 178-191 or a CD73 sequence corresponding thereto are located in the N-terminal domain of CD73, and said amino acids at positions 381-386 or a CD73 sequence corresponding thereto are located in the C-terminal domain of CD73.

33. The isolated antigen-binding protein according to any one of claims 1-32, which is capable of inhibiting the enzymatic activity of CD73.

34. The isolated antigen-binding protein according to any one of claims 1-33, which is capable of inducing endocytosis of CD73 on the cell surface.

35. A polypeptide molecule, which comprises the isolated antigen-binding protein according to any one of claims 1-34.

36. The polypeptide molecule of claim 35, which comprises a fusion protein.

37. An isolated nucleic acid molecule, which encodes the isolated antigen-binding protein according to any one of claims 1-34 or the polypeptide molecule according to any one of claims 35-36.

38. A vector, which comprises the nucleic acid molecule of claim 37.

39. A cell, which comprises the nucleic acid molecule of claim 37 and/or a vector of claim 38.

40. An immunoconjugate, which comprise the isolated antigen-binding protein according to any one of claims 1-34.

41. A pharmaceutical composition, which comprises the isolated antigen-binding protein according to any one of claims 1-34, the polypeptide molecule according to any one of claims 35-36, the said nucleic acid molecule as claimed in claim 37, the vector of claim 38, the cell of claim 39 and/or the immunoconjugate of claim 40, and optionally a pharmaceutically acceptable vector.

42. A method for preparing the isolated antigen-binding protein according to any one of claims 1-34, and said method includes culturing the said cells of claim 39 under conditions that the isolated antigen-binding protein can be expressed.

43. The use of the isolated antigen-binding protein according to any one of claims 1-34, the polypeptide molecule according to any one of claims 35-36, the nucleic acid molecule of claim 37, the vector of claim 38, the cells of claim 39, the immunoconjugate of claim 40 and/or the pharmaceutical composition of claim 41 in the manufacture of a drug and said drug is used for the prevention and/or treatment of a disease and/or disorder.

44. The use of claim 43, wherein said disease and/or disorder is a CD73-mediated disease and/or disorder.

45. The use according to any one of claims 43-44, wherein said disease and/or disorder includes a tumor.

46. The use of claim 45, wherein said tumors include a solid tumor and/or a hematological tumor.

47. The use according to any one of claims 43-46, wherein said disease and/or disorder include breast cancer.

48. A method for detecting CD73 in a sample, which includes the administration of the isolated antigen-binding protein according to any one of claims 1-34, the polypeptide molecule according to any one of claims 35-36, the nucleic acid molecule of claim 37, the vector of claim 38, the cells of claim 39, the immunoconjugate of claim 40 and/or the pharmaceutical composition of claim 41.

49. A reagent or kit for detecting CD73 in a sample, which includes the isolated antigen-binding protein according to any one of claims 1-34, the polypeptide molecule according to any one of claims 35-36, the nucleic acid molecule of claim 37, the vector of claim 38, the cells of claim 39, the immunoconjugate of claim 40 and/or the pharmaceutical composition of claim 41.

50. The use of the isolated antigen-binding protein according to any one of claims 1-34, the polypeptide molecule according to any one of claims 35-36, the nucleic acid molecule of claim 37, the vector of claim 38, the cells of claim 39, the immunoconjugate of claim 40 and/or the pharmaceutical composition of claim 41 in the manufacture of a kit and the said kit is used for the detection of the presence and/or content of CD73 in a sample.
